(19) Europäisches Patentamt
European Patent Office
Office européen des brevets

(11) Publication number: **0 166 355**
**A2**

(12) # EUROPEAN PATENT APPLICATION

(21) Application number: 85107500.2

(22) Date of filing: 18.06.85

(51) Int. Cl.[4]: **C 07 D 209/20**, C 07 C 103/50, C 07 C 125/065, C 07 K 5/00, C 07 K 5/06, C 07 K 5/08, C 07 K 5/10, C 07 D 409/12, C 07 D 333/24, C 07 D 333/38, A 61 K 37/02

(30) Priority: 26.06.84 US 624853

(43) Date of publication of application: 02.01.86 Bulletin 86/1

(84) Designated Contracting States: CH DE FR GB IT LI NL

(71) Applicant: MERCK & CO. INC., 126, East Lincoln Avenue P.O. Box 2000, Rahway New Jersey 07065 (US)

(72) Inventor: Evans, Ben E., 501 Perkiomen Avenue, Lansdale PA. 19446 (US)
Inventor: Freidinger, Roger M., 2185 Rebecca Drive, Hatfield, PA. 19440 (US)
Inventor: Bock, Mark G., 1603 Leon Drive, Hatfield, PA 19440 (US)

(74) Representative: Blum, Rudolf Emil Ernst et al, c/o E. Blum & Co Patentanwälte Vorderberg 11, CH-8044 Zürich (CH)

(54) **Substituted aminophenyl compounds and acylaminophenyl compounds and pharmaceutical compositions containing them.**

(57) Substituted aminophenyl compounds and acylaminophenyl compounds having the formula I

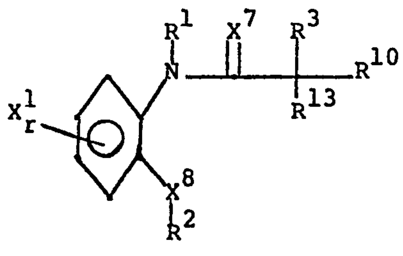

I

are disclosed. Furthermore, pharmaceutical compositions are disclosed which are useful for treating gastrointestinal disorders, central nervous system disorders, or regulating appetite in mammals.

They contain as active ingredient as antagonist of cholecystokinin (CCK), a compound of formula I or a pharmaceutically acceptable salt or quaternary ammonium salt of said compounds.

17121

TITLE OF THE INVENTION

Substituted aminophenyl compounds and acylaminophenyl compounds and pharmaceutical compositions containing them.

BACKGROUND OF THE INVENTION

Cholecystokinin (CCK) is a neuropeptide composed of thirty-three aminoacids. See: Mutt and Jorpes, Biochem. J. 125 678 (1971). The carboxyl terminal octapeptide (CCK-8) also occurs naturally and is fully active. CCK exists in both gastrointestinal tissue and the central nervous system. V. Mutt, Gastrointestinal Hormones, G. B. J. Glass, Ed., Raven Press, N.Y., p. 169. CCK is believed to play an important role in appetite regulation and CCK may be a physiological satiety hormone. G. P. Smith, Eating and Its Disorders, A. J. Stunkard and E. Stellar, Eds, Raven Press, New York, 1984, p. 67.

Among additional effects of CCK are stimulation of colonic motility, stimulation of gall bladder contraction, stimulation of pancreatic enzyme secretion, and inhibition of gastric emptying. CCK reportedly co-exists with dopamine in certain mid-brain neurons and thus may also play a role in the functioning of dopaminergic systems in the brain, as well as serving as a neurotransmitter in its own right. See: A. J. Prange et al., "Peptides in the Central Nervous System", Ann. Repts. Med. Chem. 17 31, 33 (1982) and references cited therein; J. A. Williams, Biomed. Res. 3 107 (1982); and J. E. Morley, Life Sci. 30, 479, (1982).

CCK antagonists are useful in the treatment and prevention of CCK-related disorders of the gastrointestinal, central nervous and appetite regulatory systems of animals, especially humans. Three distinct chemical classes of CCK receptor antagonists have been reported. One class comprises derivatives of cyclic nucleotides; detailed structure-function studies have demonstrated that of the various members of this class, dibutyryl cyclic GMP is the most potent. See; N. Barlos et al., Am. J. Physiol., 242, G 161 (1982) and P. Robberecht et al., Mol., Pharmacol., 17, 268 (1980). The second class comprises peptide antagonists which are C-terminal fragments and analogs of CCK. Recent structure-function studies have shown that both shorter C-terminal fragments of CCK (Boc-Met-Asp-Phe-$NH_2$, Met-Asp-Phe-$NH_2$) as well as longer CCK fragments Cbz-Tyr($SO_3H$)-Met-Gly-Trp-Met-Asp-$NH_2$) can

function as CCK antagonists. See: R. T. Jensen et al., Biochem. Biophys. Acta., 757, 250 (1983) and M. Spanarkel et al., J. Biol. Chem., 258, 6746 (1983). The third class of CCK receptor antagonists comprises the amino acid derivatives; proglumide, a derivative of glutaramic acid, and the N-acyl tryptophans including para-chlorobenzoyl-L-tryptophan (benzotript). See W. F. Hahne et al., Proc. Natl. Acad. Sci. U.S.A., 78, 6304 (1981) and R. T. Jensen et al., Biochem. Biophys. Acta., 761, 269 (1983). All of these compounds are relatively weak antagonists of CCK ($IC_{50}$: $10^{-4}$-$10^{-6}$M; generally, $10^{-4}$M but down to $10^{-6}$M in the case of peptides). The peptide antagonists have substantial stability and absorption problems.

SUMMARY OF THE INVENTION

It has now been found that compounds of Formula I are antagonists of cholecystokinin (CCK). These CCK antagonists are useful in the treatment and prevention of CCK-related disorders of the gastrointestinal, central nervous and appetite regulatory systems of mammals, especially humans. The compounds of Formula I are also gastrin antagonists. They are useful in the treatment and prevention of gastrointestinal ulcers.

DETAILED DESCRIPTION OF THE INVENTION

The compounds of this invention are those of Formula I:

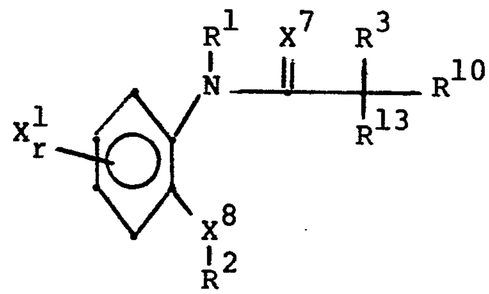

I

wherein

$R^1$ is H, $C_1$-$C_5$ linear or branched alkyl, loweralkenyl, $-(CH_2)_mCOOR^6$, $-(CH_2)_n$-cycloloweralkyl, or $-(CH_2)_mNR^4R^5$;

$R^2$ is H, loweralkyl, substituted or unsubstituted phenyl (wherein the substitutents may be 1 or 2 of halo, loweralkyl, loweralkoxy, carboxyl, carboxyloweralkyl, nitro, $-CF_3$, or hydroxy), $-(CH_2)_mSCH_3$, $-(CH_2)_mSOCH_3$, $-(CH_2)_mSO_2CH_3$, or $-(CH_2)_nCOOR^6$;

$R^3$ is $-(CH_2)_nR^7$, $-(CH_2)_n\overset{OH}{\overset{|}{C}}HR^7$, $-(CH_2)_n-\overset{OH}{\overset{|}{\underset{R^7_a}{\underset{|}{C}}}}-R^7$

$-(CH_2)_n\overset{O}{\overset{\|}{C}}R^7$, $-(CH_2)_nNH(CH_2)_qR^7$,

$-(CH_2)_mNH\underset{}{\overset{\overset{R^7}{|}}{\overset{(CH_2)_q}{\overset{|}{C}}}}HCOOR^6$, $-(CH_2)_mNH\overset{O}{\overset{\|}{C}}(CH_2)_qR^7$,

or 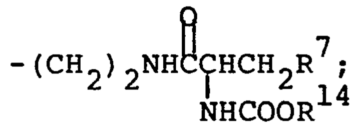

$R^4$ and $R^5$ are independently H, loweralkyl, or cycloloweralkyl;

$R^6$ is H, loweralkyl, cycloloweralkyl, substituted or unsubstituted phenyl, or substituted or unsubstituted phenylloweralkyl (wherein the substituents may be 1 or 2 of halo, loweralkyl, loweralkoxy, nitro, or $CF_3$);

$R^7$ and $R^7_a$ are independently α- or ß-naphthyl,

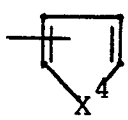 , 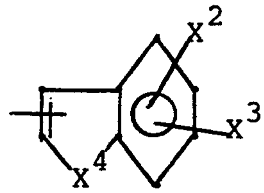 , 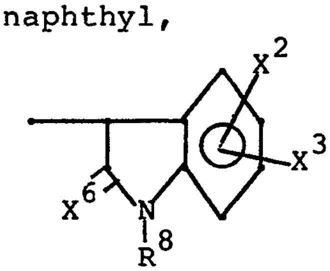

loweralkyl, substituted or unsubstituted phenyl (wherein the substituents may be 1 to 2 of halo, $-NO_2$, -OH, $-NR^4R^5$, loweralkyl, or loweralkoxy),

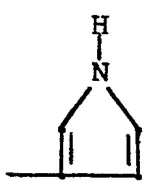 , 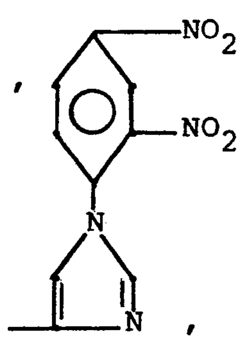 ,

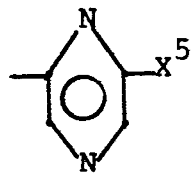

, or

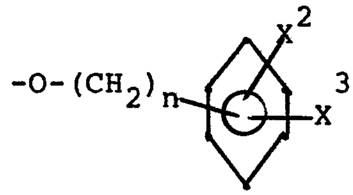

(with the proviso that q is not 1 in $-(CH_2)_nNH(CH_2)_q\ R^7$ and that q is not 0 in

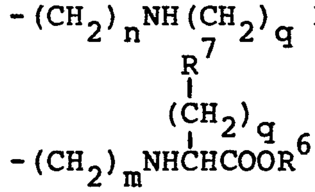

when $R^7$ or $R^7_a$ is

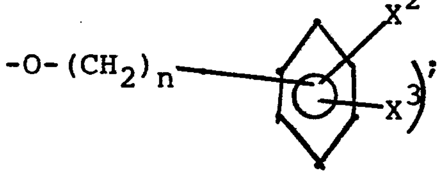

);

$R^8$ is H, loweralkyl, cycloloweralkyl,

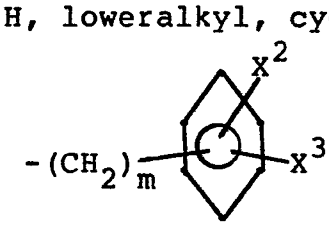

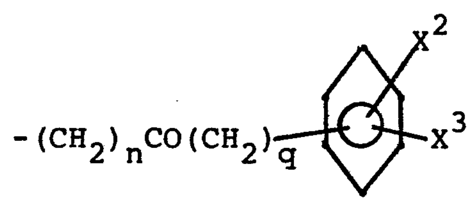

, or

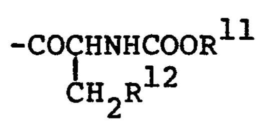

;

$R^{10}$ is H, -OH, or $-CH_3$;

$R^{11}$ and $R^{12}$ are independently loweralkyl or cycloloweralkyl;

$R^{13}$ is H, or $-NH\overset{O}{\overset{\|}{C}}R^{16}$;

$R^{14}$ is loweralkyl or phenylloweralkyl;

$R^{16}$ is H, loweralkyl, $-O(CH_2)_n$ 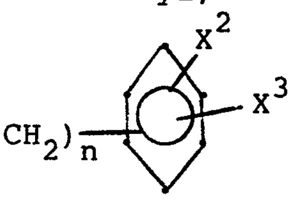 ,

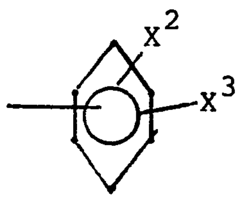 , 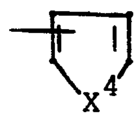 ,

$-(CH_2)_nNHCOOR^{11}$, $-(CH_2)_nNHCO\underset{NHCOOR^{11}}{\overset{H}{C}}(CH_2)_nX^4R^{12}$,

$OR^{11}$, or $-(CH_2)_m\overset{H}{N}R^{17}$;

$R^{17}$ is $A_1A_2A_3$-, $A_1A_3$-, or $A_1$-;

where

$A_1$ is H, Boc, Cbz, or acetyl;

$A_2$ is Tyr, Tyr-O-sulfate or phenyl;

$A_3$ is Met, nor-Leu, or nor-Val;

m is 1-4;

n is 0-4;

q is 0-4;

r is 1 or 2;

$X^1$ is H, $-NO_2$, $CF_3$ CN, OH, loweralkyl, halo, loweralkylthio, loweralkoxy, $-(CH_2)_nCOOR^6$, or $-NR^4R^5$;

$X^2$ and $X^3$ are independently H, -OH, $-NO_2$, halo, loweralkylthio, loweralkyl, or loweralkoxy;

$X^4$ is S, O, or $NR^8$;

$X^5$ is H, $CF_3$, CN, $COOR^6$, $NO_2$, or halo;

$X^6$ is O or HH;

$X^7$ is O, or HH;

$X^8$ is C=O, $-NR^{10}$, or $-\overset{\text{OH}}{\overset{|}{\text{C}}}H_2R^2$;

and the pharmaceutically acceptable salts thereof.

In the compounds of Formula I, the preferred stereochemistry relates to L-tryptophan, where $C=X^7$, $R^{13}$, and $R^3$ of Formula I occupy the positions of the carbonyl group, the α-amino group, and the indolymethyl side chain, respectively, of L-tryptophan.

As used herein, the definition of each expression, e.g. m, n, loweralkyl, etc., when it occurs more than once in any structure, is intended to be independent of its definiton elsewhere in the same structure.

As used herein, halo is F, Cl, or Br; loweralkyl is 1-4 carbon straight or branched chain alkyl and includes methyl, ethyl, propyl, isopropyl, butyl, isobutyl, and t-butyl; in loweralkoxy and loweralkylthio, the alkyl portion is loweralkyl as previously defined; cycloloweralkyl is cycloalkyl of 3-5 carbons; loweralkenyl is 1-5 carbon straight or branched chain alkenyl; and acyl is formyl, acetyl propionyl, or butyryl.

The pharmaceutically acceptable salts of the compounds of Formulas I include the conventional non-toxic salts or the quarternary ammonium salts of the compounds of Formula I formed, e.g., from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, theophylline, 8-chlorotheophylline, p-aminobenzoic, p-acetamidobenzoic, methanesulfonic, ethane disulfonic, oxalic, isethionic, and the like.

The pharmaceutically acceptable salts of the present invention can be synthesized from the compounds of Formula I which contain a basic or acidic moiety by conventional chemical methods. Generally, the salts are prepared by reacting the free base or acid with stoichiometric amounts or with an excess of the desired salt forming inorganic or organic acid or base in a suitable solvent or various combinations of solvents.

The pharmaceutically acceptable salts of the acid of Formula I are also readily prepared by conventional procedures such as treating an acid of Formula I with an appropriate amount of a base, such as an alkali or alkaline earth metal hydroxide e.g.

sodium, potassium, lithium, calcium, or magnesium, or an organic base such as an amine, e.g., dibenzylethylenediamine, trimethylamine, piperidine, pyrrolidine, benzylamine and the like, or a quaternary ammonium hydroxide such as tetramethylammonium hydroxide and the like.

An embodiment of this invention is the preparation of compounds of Formula I.

Another embodiment is the use of the compounds of Formula I for the treatment and the prevention of disorders of the gastrointestinal, central nervous, and appetite regulatory systems of mammals, especially of man. Specifically, the Formula I compounds are useful in treatment and prevention of disorders of gastric acid secretion, gastrointestinal motility, pancreatic secretions, and dopaminergic functions. The compounds of Formula I are especially useful in the prevention and treatment of irritable bowel syndrome.

A further embodiment is a composition comprising an effective amount of a compound of Formula I and a pharmaceutically acceptable carrier.

The ability of the compounds of Formula I to antagonize CCK and gastrin makes these compounds useful as pharmaceutical agents. These compounds will be especially useful in the treatment and prevention of disease states wherein CCK or gastrin may be involved, for example, gastrointestinal disorders such as irritable bowel syndrome, ulcers, excess pancreatic or gastric secretion, acute pancreatitis, motility disorders, central nervous

system disorders caused by CCK's interaction with dopamine such as neuroleptic disorders, tardive dyskinesia, Parkinson's disease, psychosis or Gilles de la Tourette Syndrome, and disorders of appetite regulatory systems.

The compounds of Formula I or pharmaceutically acceptable salts thereof, can be administered to a human subject either alone, or preferably, in combination with pharmaceutically acceptable carriers or diluents, in a pharmaceutical composition, according to standard pharmaceutical practice. The compounds can be administered orally or parenterally. Parenteral administration includes intravenous, intramuscular, intraperitoneal, subcutaneous and topical administration.

For oral use of an antagonist of CCK or gastrin of this invention, the selected compound can be administered, for example, in the form of tablets or capsules, or as an aqueous solution or suspension. In the case of tablets for oral use, carriers which are commonly used include lactose and corn starch, and lubricating agents, such as magnesium stearate, are commonly added. For oral administration in capsule form, useful diluents are lactose and dried corn starch. When aqueous suspensions are required for oral use, the active ingredient is combined with emulsifying and suspending agents. If desired, certain sweetening and/or flavoring agents can be added. For intramuscular, intraperitoneal, subcutaneous and intravenous use, sterile solutions of the active

ingredient are usually prepared, and the pH of the solutions should be suitably adjusted and buffered. For intravenous use, the total concentration of solutes should be controlled to render the preparation isotonic.

When a compound of Formula I or a salt thereof is used as an antagonist of CCK or gastrin in a human subject, the daily dosage will normally be determined by the prescribing physician. Moreover, the dosage will vary according to the age, weight, and response of the individual patient, as well as the severity of the patient's symptoms. However, in most instances, an effective daily dosage will be in the range from about 0.05 mg to about 50 mg/kg and preferably 0.5 mg to about 20 mg/kg in a single or divided doses. On the other hand, it may be necessary to use dosages outside these limits in some cases.

The compounds of Formula I are prepared according to the following schemes.

REACTION SCHEME I

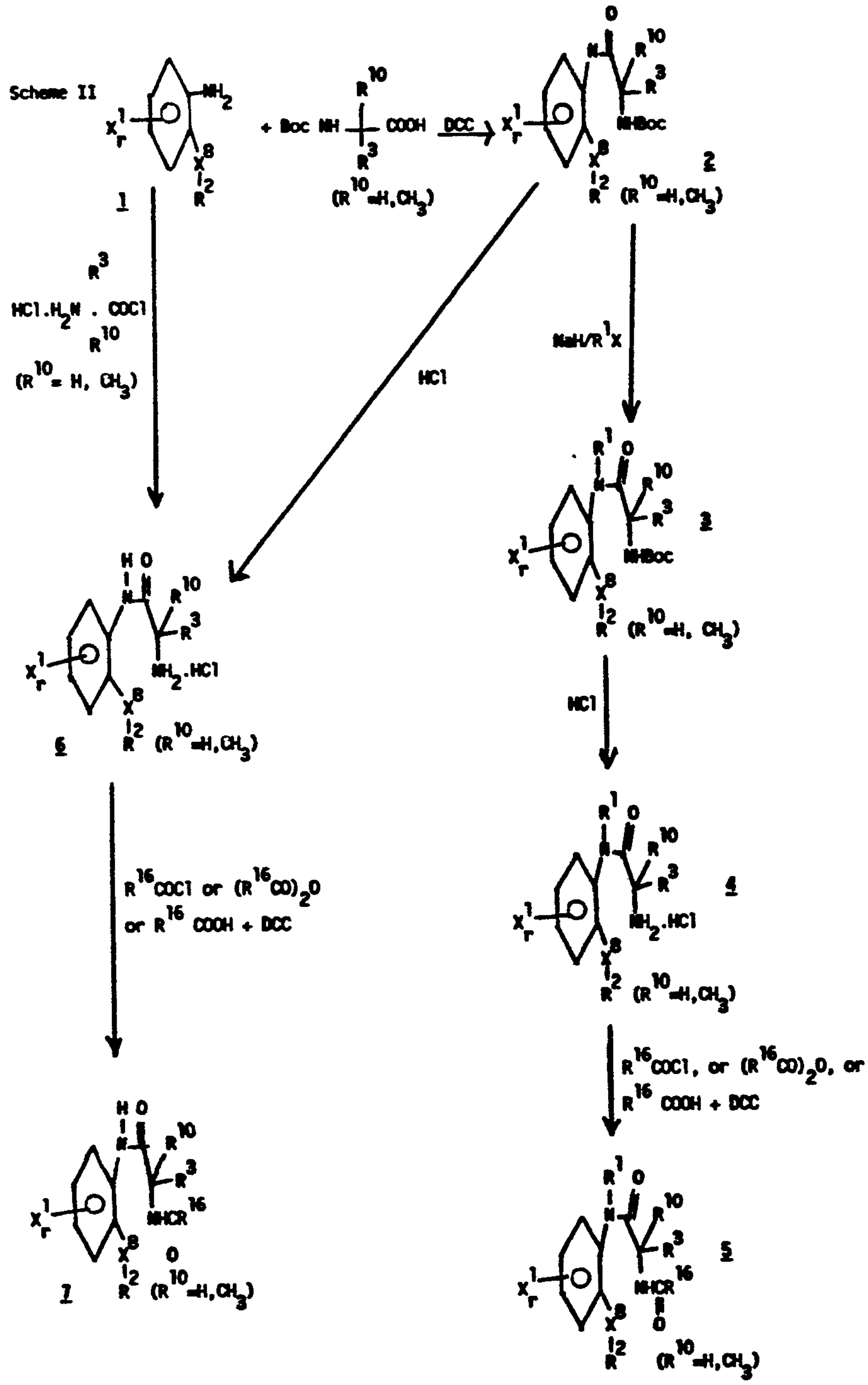

REACTION SCHEME II

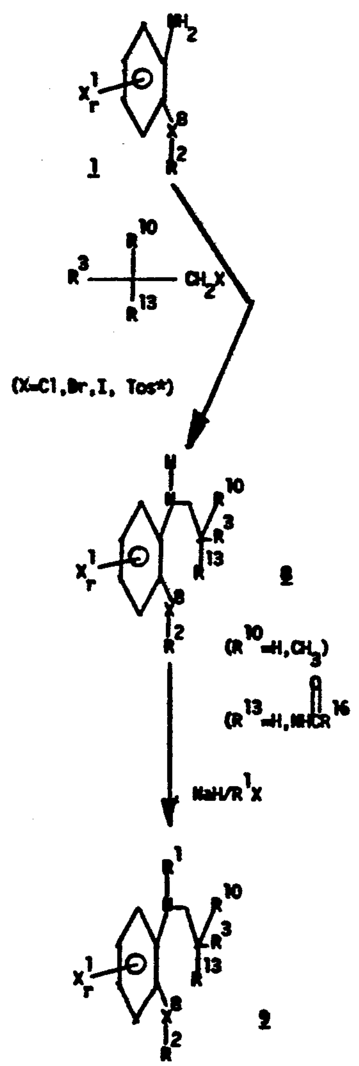


* paratoluenesulfonate ester

REACTION SCHEME III

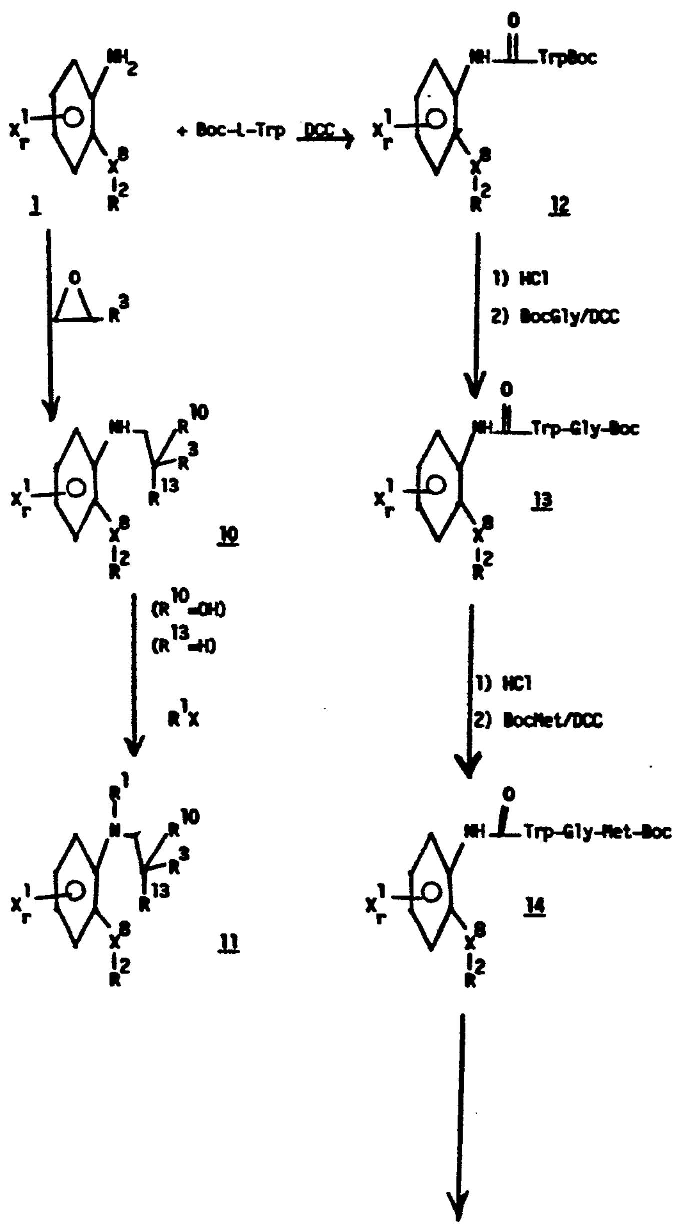

REACTION SCHEME III (cont'd)

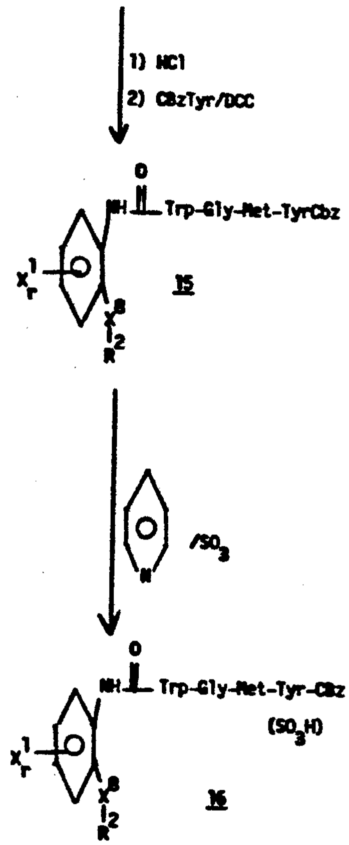


15

16

REACTION SCHEME IV

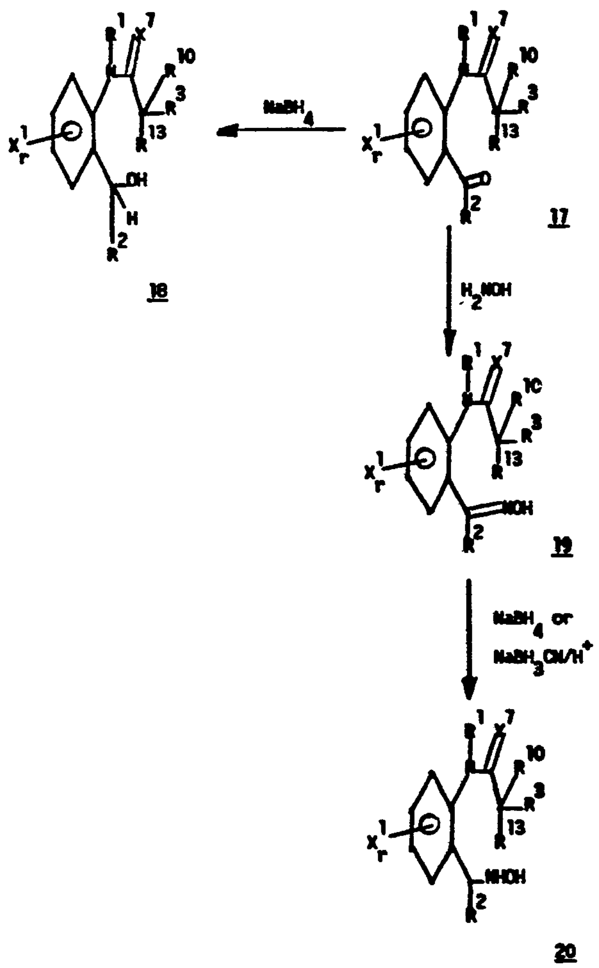

2-Aminoarylketones (1 where $X^8$= C=0), preferably 2-aminobenzophenones, 1-amino-2-N-substituted aminobenzenes (1 where $X^8 = NR_{10}$), preferably 1-amino-2-arylaminobenzenes, or 2-substituted anilines (1 where $R^8 = CH_2R^2$), preferably 2-(substituted phenylmethyl)anilines, each containing various substituents in the aryl rings, preferably halo substituents, are coupled to N-protected L-amino acids preferably Boc-L-amino acids, using dicyclohexylcarbodiimides (DCC), or other conventional peptide coupling reagent, to give the amides 2. 2 may be deprotected, preferably with HCl, to give the amines 6 and these may be akylated by treatment with alkyl halide or dialkyl sulfate or acylated by treatment with acyl halides or anhydrides, preferably in the presence of a base such as triethylamine or the like, to give the alkyl or acyl derivative 7. Alternatively, 6 may be obtained from 1 directly by acylation with an L-amino acid chloride hydrochloride in the presence or absence of a base such as triethylamine.

The N-protected compounds 2 may first be alkylated on the aniline nitrogen by treatment with sodium hydride in dimethylformamide (DMF), followed by an alkyl halide, to give the alkyl derivatives 3. These may be deprotected as described for 2 above to give the amines 4 which may be alkylated or acylated, again as described above, to give 5.

Alternatively, compounds 1 may be reacted with an alkyl halide or sulfonate ester to give the amines 8 which may also be alkylated at the aniline nitrogen, as described for 2 above, to give the compounds 9.

Alternatively, compounds 1 may be treated with an epoxide to give the carbinolamines 10 which may also be alkylated at the aniline nitrogen to give the compounds 11.

When compounds 1 are coupled to an N-protected L-amino acid which is Boc-L-tryptophan, the product 2 obtained is that shown by structure 12. Deprotection with HCl and coupling with Boc-Gly using DCC, converts 12 to 13. Similar deprotection and coupling of 13 with Boc-L-Met gives 14, and another deprotection and coupling with Cbz-L-Tyr provides 15. Treatment of 15 with pyridine sulfur trioxide complex or other sulfating reagent gives the O-sulfate 16.

Reduction of ketones 17 with sodium borohydride gives the carbinols 18. Alternatively, treatment of 17 with hydroxylamine provides the oximes 19 which are reduced with sodium cyanoborohydride in acidic medium to the hydroxylamines 20.

In cases where the starting materials are optically active, the chirality at the carbon bearing $R^3$, $R_{10}$, and $R_{13}$ is controlled by the synthesis. When racemic starting materials are employed, racemic products are obtained. The enantiomers may be separated by resolution.

In Vitro Activity of Formula I

The biological activity of the compounds of Formula I have been evaluated using an $^{125}I$-CCK receptor binding assay and *in vitro* isolated tissue preparations.

Materials and Methods

1. CCK Receptor Binding (Pancreas)

CCK-33 was radiolabeled with $^{125}$I-Bolton Hunter reagent (2000 Ci/mmole) as described by Sankara et al. (J. Biol. Chem. 254: 9349-9351, 1979). Receptor binding was performed according to Innis and Snyder (Proc. Natl. Acad. Sci. 77: 6917-6921, 1980) with the minor modification of adding the additional protease inhibitors, phenylmethane sulfonyl fluoride and o-phenanthroline. The latter two compounds have no effect on the $^{125}$I-CCK receptor binding assay.

Male Sprague-Dawley rats (200-350g) were sacrificed by decapitation. The whole pancreas was dissected free of fat tissue and was homogenized in 20 volumes of ice-cold 50 mM, Tris HCl (pH 7.7 at 25°C) with a Brinkmann Polytron PT 10. The homogenates were centrifuged at 48,000 g for 10 min. Pellets were resuspended in Tris Buffer, centrifuged as above and resuspended in 200 volumes of binding assay buffer (50 mM Tris HCl, pH 7.7 at 25°C, 5 mM dithiothrietol, 0.1 mM bacitracin, 1.2 mM phenylmethane sulfonyl fluoride and 0.5 mM o-phenanthroline). For the binding assay, 25 µl of buffer (for total binding) or unlabeled CCK-8 sulfate to give a final concentration of 1µM (for nonspecific binding) or the compounds of Formula I (for determination inhibition of $^{125}$I-CCK binding) and 25 µl of $^{125}$I-CCK-33 (30,000-40,000 cpm) were added to 450 µl of the membrane suspensions in microfuge tubes. All assays were run in duplicate or triplicate. The reaction mixtures were incubated at 37°C for 30 minutes and centrifuged in a Beckman

Microfuge (4 minutes) immediately after adding 1 ml of ice-cold incubation buffer. The supernatant was aspirated and discarded, pellets were counted with a Beckman gamma 5000. For Scatchard analysis (Ann. N.Y. Acad. Sci. 51: 660, 1949), $^{125}$I-CCK-33 was progressively diluted with increasing concentrations of CCK-33.

2. CCK Receptor Binding (Brain)

CCK-33 was radiolabeled and the binding was performed according to the description for the pancreas method with modifications according to Saito et al., J. Neurochem., 37 483-490, 1981.

Male Hartley guinea pigs (300-500g) were sacrificed by decapitation and the brains were removed and placed in ice-cold 50 mM, Tris HCl plus 7.58 g/l Trizma-7.4 (pH 7.4 at 25°C) Cerebral cortex was dissected and used as a receptor source. Each gram of fresh guinea pig brain tissue was homogenized in 10 ml of Tris Trizma buffer with a Brinkman polytron PT-10. The homogenates were centrifuged at 42,000 g for 15 min. Pellets were resuspended in Tris Buffer, centrifuged as above and resuspended in 200 volumes of binding assay buffer (10 mM N-2-hydroxethyl-piperazine-N'-2-ethane sulfonic acid (HEPES), 5 mM $MgCl_2$, 0.25 mg/ml bacitracin, 1 mM ethylene glycol-bis-(ß-aminoethyl-ether-N,N'-tetraacetic acid (EGTA), and 0.4% bovine serum albumin (BSA)). For the binding assay, 25 µl of buffer (for total binding) or unlabeled CCK-8 sulfate to give a final concentration of 1µM (for nonspecific binding) or the compounds of Formula I (for determination inhibition of $^{125}$I-CCK binding) and 25 µl of $^{125}$I-CCK-33

(30,000-40,000 cpm) were added to 450 µl of the membrane suspensions in microfuge tubes. All assays were run in duplicate or triplicate. The reaction mixtures were incubated at 25°C for 2 hours and centrifuged in a Beckman Microfuge (4 minutes) immediately after adding 1 ml of ice-cold incubation buffer. The supernatant was aspirated and discarded, pellets were counted with a Beckman gamma 5000.

The compounds of Formula I can be determined to be competitive antagonists of CCK according to the following assays.

3. Isolated guinea pig gall bladder

Male Hartley guinea pigs (400-600 g) are sacrificed by decapitation. The whole gall bladder is dissected free from adjacent tissues and cut into two equal halves. The gall bladder strips are suspended along the axis of bile duct in 5 ml organ bath under 1 g tension. The organ bath contains a Kreb's bicarbonate solution (NaCl 118 mM, KCl 4.75 mM, CaCl 2.54 mM, $KH_2PO_4$ 1.19 mM, Mg $SO_4$ 1.2 mM, $NaHCO_3$ 25 mM and dextrose 11 mM) maintained at 32°C and bubbled with 95% $O_2$ and 5% $CO_2$. Isometric contractions are recorded using Statham (60 g; 0.12 mm) strain gauges and a Hewlett-Packard (77588) recorder. The tissues are washed every 10 minutes for 1 hr to obtain equilibrium prior to the beginning of the study. CCK-8 is added cumulatively to the baths and $EC_{50}$'s determined using regression analysis. After washout (every 10 minutes for 1 hr), the compound of Formula I is added at least 5 minutes before the addition of CCK-8 and the $EC_{50}$ of CCK-8 in the presence of the compound of Formula I similarly determined.

4. Isolated longitudinal muscle of guinea pig ileum

Longitudinal muscle strips with attached nerve plexus are prepared as described in Brit J. Pharmac. 23: ; 356-363, 1964; J. Physiol. 194: 13-33, 1969. Male Hartley guinea pigs are decapitated and the ileum is removed (10 cm of the terminal ileum is discarded and the adjacent 20 cm piece is used). A piece (10 cm) of the ileum is stretched on a glass pipette. Using a cotton applicator to stroke tangently away from the mesentery attachment at one end, the longitudinal muscle is separated from the underlying circular muscle. The longitudinal muscle is then tied to a thread and by gently pulling, stripped away from the entire muscle. A piece of approximately 2 cm is suspended in 5 ml organ bath containing Krebs solution and bubbled with 95% $O_2$ and 5% $CO_2$ at 37°C under 0.5 g tension. CCK-8 is added cumulatively to the baths and $EC_{50}$ values in the presence and absence of compounds of Formula I determined as described in the gall bladder protocol (above).

In Vitro Results

1. Effect of The Compounds of Formula I on $^{125}$I-CCK-33 receptor binding

The preferred compounds of Formula I are those which inhibited specific $^{125}$I-CCK-33 binding in a concentration dependent manner.

The data of Table 1 were obtained for compounds of Formula I:

TABLE 1

Receptor Binding Results

| | $IC_{50}$ (μM) |
|---|---|
| Compound of Example | $^{125}$I-CCK Pancreas |
| 2 | 0.74 |
| 3 | 3.70 |
| 5 | 4.80 |
| 12 | 1.30 |
| 13 | 10.00 |
| 15 | 0.90 |
| 16 | 1.00 |
| 17 | 0.19 |
| 18 | 8.00 |
| 19 | 2.40 |
| 20 | 2.00 |
| 21 | 0.40 |
| 22B | 2.20 |

Preferred compounds of Formula I are those where $R^1$ is H, methyl, ethyl, carboxymethyl, or carboxyethyl.

Other series of preferred compounds are those wherein $R^2$ is phenyl, p-chlorophenyl, o-fluorophenyl, 2,4-dichlorophenyl, 2,6-difluorophenyl, $-CH_2COO$-t-butyl, or $-CH_2COOEt$.

It is preferred that $R^3$ is $-(CH_2)_{1-3}$-2 or 3-indolyl, or $-(CH_2)_{0-3}$-2 or 3-thiophene, that $R^{10}$ is H, that X is O or HH, and that $X^8$ is -C=O.

It is also preferred tht $X_r^1$ is H, Cl, F, $CF_3$, or $NO_2$.

$R^{13}$ is preferably $-NHCOOC(CH_3)_3$, $-NHCOOCH_2Ph$, or -NHCO-2 or 3-thiophene.

Examples of Formula I compounds are tabulated below.

**TABLE 2**

Compounds of the Formula:

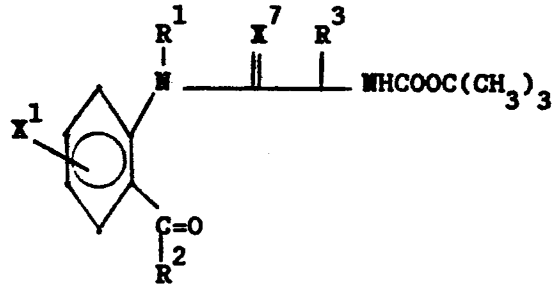

| $X^1$ | $R^1$ | $R^2$ | $R^3$ | $X^7$ |
|---|---|---|---|---|
| H | H | Ph | $-(CH_2)_{1-3}$-2-indolyl | O |
| H | H | Ph | $-(CH_2)_{1-3}$-3-indolyl | O |
| H | H | p-Cl-Ph | $-(CH_2)_{1-3}$-2-indolyl | O |
| H | H | p-Cl-Ph | $-(CH_2)_{1-3}$-3-indolyl | O |
| H | H | o-F-Ph | $-(CH_2)_{1-3}$-2-indolyl | O |
| H | H | o-F-Ph | $-(CH_2)_{1-3}$-3-indolyl | O |
| H | H | 2,4-di-Cl-Ph | $-(CH_2)_{1-3}$-2-indolyl | O |
| H | H | 2,4-di-Cl-Ph | $-(CH_2)_{1-3}$-3-indolyl | O |
| H | H | 2,6-di-F-Ph | $-(CH_2)_{1-3}$-2-indolyl | O |
| H | H | 2,6-di-F-Ph | $-(CH_2)_{1-3}$-3-indolyl | O |
| H | H | $-CH_2COO$-t-Bu | $-(CH_2)_{1-3}$-2-indolyl | O |
| H | H | $-CH_2COO$-t-Bu | $-(CH_2)_{1-3}$-3-indolyl | O |
| H | H | $-CH_2COOEt$ | $-(CH_2)_{1-3}$-2-indolyl | O |
| H | H | $-CH_2COOEt$ | $-(CH_2)_{1-3}$-3-indolyl | O |
| H | $CH_3$ | Ph | $-(CH_2)_{1-3}$-2-indolyl | O |
| H | $CH_3$ | p-Cl-Ph | $-(CH_2)_{1-3}$-2-indolyl | O |
| H | $CH_3$ | o-F-Ph | $-(CH_2)_{1-3}$-2-indolyl | O |
| H | $CH_3$ | 2,4-di-Cl-Ph | $-(CH_2)_{1-3}$-2-indolyl | O |
| H | $CH_3$ | 2,6-di-F-Ph | $-(CH_2)_{1-3}$-2-indolyl | O |
| H | $CH_3$ | $-CH_2COO$-t-Bu | $-(CH_2)_{1-3}$-2-indolyl | O |

TABLE 2 (Cont'd)

| $X^1$ | $R^1$ | $R^2$ | $R^3$ | $X^7$ |
|---|---|---|---|---|
| H | $CH_3$ | $-CH_2COOEt$ | $-(CH_2)_{1-3}$-2-indolyl | O |
| H | $-CH_2COOH$ | Ph | $-(CH_2)_{1-3}$-3-indolyl | O |
| H | $-CH_2COOH$ | p-Cl-Ph | $-(CH_2)_{1-3}$-3-indolyl | O |
| H | $-CH_2COOH$ | o-F-Ph | $-(CH_2)_{1-3}$-3-indolyl | O |
| H | $-CH_2COOH$ | 2,4-di-Cl-Ph | $-(CH_2)_{1-3}$-3-indolyl | O |
| H | $-CH_2COOH$ | 2,6-di-F-Ph | $-(CH_2)_{1-3}$-3-indolyl | O |
| H | $-CH_2COOH$ | $-CH_2COO$-t-Bu | $-(CH_2)_{1-3}$-3-indolyl | O |
| H | $-CH_2COOH$ | $-CH_2COOEt$ | $-(CH_2)_{1-3}$-3-indolyl | O |
| Cl | H | Ph | $-(CH_2)_{1-3}$-2-indolyl | O |
| Cl | H | Ph | $-(CH_2)_{1-3}$-3-indolyl | O |
| Cl | H | p-Cl-Ph | $-(CH_2)_{1-3}$-2-indolyl | O |
| Cl | H | p-Cl-Ph | $-(CH_2)_{1-3}$-3-indolyl | O |
| Cl | H | o-F-Ph | $-(CH_2)_{1-3}$-2-indolyl | O |
| Cl | H | o-F-Ph | $-(CH_2)_{1-3}$-3-indolyl | O |
| Cl | H | 2,4-di-Cl-Ph | $-(CH_2)_{1-3}$-2-indolyl | O |
| Cl | H | 2,4-di-Cl-Ph | $-(CH_2)_{1-3}$-3-indolyl | O |
| Cl | H | 2,6-di-F-Ph | $-(CH_2)_{1-3}$-2-indolyl | O |
| Cl | H | 2,6-di-F-Ph | $-(CH_2)_{1-3}$-3-indolyl | O |
| Cl | H | $-CH_2COO$-t-Bu | $-(CH_2)_{1-3}$-2-indolyl | O |
| Cl | H | $-CH_2COO$-t-Bu | $-(CH_2)_{1-3}$-3-indolyl | O |
| Cl | H | $-CH_2COOEt$ | $-(CH_2)_{1-3}$-2-indolyl | O |
| Cl | H | $-CH_2COOEt$ | $-(CH_2)_{1-3}$-3-indolyl | O |
| Cl | $CH_3$ | Ph | $-(CH_2)_{1-3}$-2-indolyl | O |
| Cl | $CH_3$ | p-Cl-Ph | $-(CH_2)_{1-3}$-2-indolyl | O |
| Cl | $CH_3$ | o-F-Ph | $-(CH_2)_{1-3}$-2-indolyl | O |
| Cl | $CH_3$ | 2,4-di-Cl-Ph | $-(CH_2)_{1-3}$-2-indolyl | O |
| Cl | $CH_3$ | 2,6-di-F-Ph | $-(CH_2)_{1-3}$-2-indolyl | O |
| Cl | $CH_3$ | $-CH_2COO$-t-Bu | $-(CH_2)_{1-3}$-2-indolyl | O |

TABLE 2 (Cont'd)

| $X^1$ | $R^1$ | $R^2$ | $R^3$ | $X^7$ |
|---|---|---|---|---|
| Cl | $CH_3$ | $-CH_2COOEt$ | $-(CH_2)_{1-3}$-2-indolyl | O |
| Cl | $-CH_2COOH$ | Ph | $-(CH_2)_{1-3}$-3-indolyl | O |
| Cl | $-CH_2COOH$ | p-Cl-Ph | $-(CH_2)_{1-3}$-3-indolyl | O |
| Cl | $-CH_2COOH$ | o-F-Ph | $-(CH_2)_{1-3}$-3-indolyl | O |
| Cl | $-CH_2COOH$ | 2,4-di-Cl-Ph | $-(CH_2)_{1-3}$-3-indolyl | O |
| Cl | $-CH_2COOH$ | 2,6-di-F-Ph | $-(CH_2)_{1-3}$-3-indolyl | O |
| Cl | $-CH_2COOH$ | $-CH_2COO$-t-Bu | $-(CH_2)_{1-3}$-3-indolyl | O |
| Cl | $-CH_2COOH$ | $-CH_2COOEt$ | $-(CH_2)_{1-3}$-3-indolyl | O |
| F | H | Ph | $-(CH_2)_{1-3}$-2-indolyl | O |
| F | H | Ph | $-(CH_2)_{1-3}$-3-indolyl | O |
| F | H | p-Cl-Ph | $-(CH_2)_{1-3}$-2-indolyl | O |
| F | H | p-Cl-Ph | $-(CH_2)_{1-3}$-3-indolyl | O |
| F | H | o-F-Ph | $-(CH_2)_{1-3}$-2-indolyl | O |
| F | H | o-F-Ph | $-(CH_2)_{1-3}$-3-indolyl | O |
| F | H | 2,4-di-Cl-Ph | $-(CH_2)_{1-3}$-2-indolyl | O |
| F | H | 2,4-di-Cl-Ph | $-(CH_2)_{1-3}$-3-indolyl | O |
| F | H | 2,6-di-F-Ph | $-(CH_2)_{1-3}$-2-indolyl | O |
| F | H | 2,6-di-F-Ph | $-(CH_2)_{1-3}$-3-indolyl | O |
| F | H | $-CH_2COO$-t-Bu | $-(CH_2)_{1-3}$-2-indolyl | O |
| F | H | $-CH_2COO$-t-Bu | $-(CH_2)_{1-3}$-3-indolyl | O |
| F | H | $-CH_2COOEt$ | $-(CH_2)_{1-3}$-2-indolyl | O |
| F | H | $-CH_2COOEt$ | $-(CH_2)_{1-3}$-3-indolyl | O |
| F | $CH_3$ | Ph | $-(CH_2)_{1-3}$-2-indolyl | O |
| F | $CH_3$ | p-Cl-Ph | $-(CH_2)_{1-3}$-2-indolyl | O |
| F | $CH_3$ | o-F-Ph | $-(CH_2)_{1-3}$-2-indolyl | O |
| F | $CH_3$ | 2,4-di-Cl-Ph | $-(CH_2)_{1-3}$-2-indolyl | O |
| F | $CH_3$ | 2,6-di-F-Ph | $-(CH_2)_{1-3}$-2-indolyl | O |
| F | $CH_3$ | $-CH_2COO$-t-Bu | $-(CH_2)_{1-3}$-2-indolyl | O |

TABLE 2 (Cont'd)

| $X^1$ | $R^1$ | $R^2$ | $R^3$ | $X^7$ |
|---|---|---|---|---|
| F | $CH_3$ | $-CH_2COOEt$ | $-(CH_2)_{1-3}$-2-indolyl | O |
| F | $-CH_2COOH$ | Ph | $-(CH_2)_{1-3}$-3-indolyl | O |
| F | $-CH_2COOH$ | p-Cl-Ph | $-(CH_2)_{1-3}$-3-indolyl | O |
| F | $-CH_2COOH$ | o-F-Ph | $-(CH_2)_{1-3}$-3-indolyl | O |
| F | $-CH_2COOH$ | 2,4-di-Cl-Ph | $-(CH_2)_{1-3}$-3-indolyl | O |
| F | $-CH_2COOH$ | 2,6-di-F-Ph | $-(CH_2)_{1-3}$-3-indolyl | O |
| F | $-CH_2COOH$ | $-CH_2COO$-t-Bu | $-(CH_2)_{1-3}$-3-indolyl | O |
| F | $-CH_2COOH$ | $-CH_2COOEt$ | $-(CH_2)_{1-3}$-3-indolyl | O |
| $CF_3$ | $CH_3$ | Ph | $-(CH_2)_{1-3}$-2-indolyl | O |
| $CF_3$ | $CH_3$ | p-Cl-Ph | $-(CH_2)_{1-3}$-2-indolyl | O |
| $CF_3$ | $CH_3$ | o-F-Ph | $-(CH_2)_{1-3}$-2-indolyl | O |
| $CF_3$ | $CH_3$ | 2,4-di-Cl-Ph | $-(CH_2)_{1-3}$-2-indolyl | O |
| $CF_3$ | $CH_3$ | 2,6-di-F-Ph | $-(CH_2)_{1-3}$-2-indolyl | O |
| $CF_3$ | $CH_3$ | $-CH_2COO$-t-Bu | $-(CH_2)_{1-3}$-2-indolyl | O |
| $CF_3$ | $CH_3$ | $-CH_2COOEt$ | $-(CH_2)_{1-3}$-2-indolyl | O |
| $CF_3$ | $CH_2CH_3$ | Ph | $-(CH_2)_{1-3}$-3-indolyl | O |
| $CF_3$ | $CH_2CH_3$ | p-Cl-Ph | $-(CH_2)_{1-3}$-3-indolyl | O |
| $CF_3$ | $CH_2CH_3$ | o-F-Ph | $-(CH_2)_{1-3}$-3-indolyl | O |
| $CF_3$ | $CH_2CH_3$ | 2,4-di-Cl-Ph | $-(CH_2)_{1-3}$-3-indolyl | O |
| $CF_3$ | $CH_2CH_3$ | 2,6-di-F-Ph | $-(CH_2)_{1-3}$-3-indolyl | O |
| $CF_3$ | $CH_2CH_3$ | $-CH_2COO$-t-Bu | $-(CH_2)_{1-3}$-3-indolyl | O |
| $CF_3$ | $CH_2CH_3$ | $-CH_2COOEt$ | $-(CH_2)_{1-3}$-3-indolyl | O |
| $CF_3$ | $-(CH_2)_2COOH$ | Ph | $-(CH_2)_{1-3}$-2-indolyl | O |
| $CF_3$ | $-(CH_2)_2COOH$ | p-Cl-Ph | $-(CH_2)_{1-3}$-2-indolyl | O |
| $CF_3$ | $-(CH_2)_2COOH$ | o-F-Ph | $-(CH_2)_{1-3}$-2-indolyl | O |
| $CF_3$ | $-(CH_2)_2COOH$ | 2,4-di-Cl-Ph | $-(CH_2)_{1-3}$-2-indolyl | O |
| $CF_3$ | $-(CH_2)_2COOH$ | 2,6-di-F-Ph | $-(CH_2)_{1-3}$-2-indolyl | O |
| $CF_3$ | $-(CH_2)_2COOH$ | $-CH_2COO$-t-Bu | $-(CH_2)_{1-3}$-2-indolyl | O |
| $CF_3$ | $-(CH_2)_2COOH$ | $-CH_2COOEt$ | $-(CH_2)_{1-3}$-2-indolyl | O |

TABLE 2 (Cont'd)

| $X^1$ | $R^1$ | $R^2$ | $R^3$ | $X^7$ |
|---|---|---|---|---|
| $NO_2$ | $CH_3$ | Ph | $-(CH_2)_{1-3}$-2-indolyl | O |
| $NO_2$ | $CH_3$ | p-Cl-Ph | $-(CH_2)_{1-3}$-2-indolyl | O |
| $NO_2$ | $CH_3$ | o-F-Ph | $-(CH_2)_{1-3}$-2-indolyl | O |
| $NO_2$ | $CH_3$ | 2,4-di-Cl-Ph | $-(CH_2)_{1-3}$-2-indolyl | O |
| $NO_2$ | $CH_3$ | 2,6-di-F-Ph | $-(CH_2)_{1-3}$-2-indolyl | O |
| $NO_2$ | $CH_3$ | $-CH_2COO$-t-Bu | $-(CH_2)_{1-3}$-2-indolyl | O |
| $NO_2$ | $CH_3$ | $-CH_2COOEt$ | $-(CH_2)_{1-3}$-2-indolyl | O |
| $NO_2$ | $CH_2CH_3$ | Ph | $-(CH_2)_{1-3}$-3-indolyl | O |
| $NO_2$ | $CH_2CH_3$ | p-Cl-Ph | $-(CH_2)_{1-3}$-3-indolyl | O |
| $NO_2$ | $CH_2CH_3$ | o-F-Ph | $-(CH_2)_{1-3}$-3-indolyl | O |
| $NO_2$ | $CH_2CH_3$ | 2,4-di-Cl-Ph | $-(CH_2)_{1-3}$-3-indolyl | O |
| $NO_2$ | $CH_2CH_3$ | 2,6-di-F-Ph | $-(CH_2)_{1-3}$-3-indolyl | O |
| $NO_2$ | $CH_2CH_3$ | $-CH_2COO$-t-Bu | $-(CH_2)_{1-3}$-3-indolyl | O |
| $NO_2$ | $CH_2CH_3$ | $-CH_2COOEt$ | $-(CH_2)_{1-3}$-3-indolyl | O |
| $NO_2$ | $-(CH_2)_2COOH$ | Ph | $-(CH_2)_{1-3}$-2-indolyl | O |
| $NO_2$ | $-(CH_2)_2COOH$ | p-Cl-Ph | $-(CH_2)_{1-3}$-2-indolyl | O |
| $NO_2$ | $-(CH_2)_2COOH$ | o-F-Ph | $-(CH_2)_{1-3}$-2-indolyl | O |
| $NO_2$ | $-(CH_2)_2COOH$ | 2,4-di-Cl-Ph | $-(CH_2)_{1-3}$-2-indolyl | O |
| $NO_2$ | $-(CH_2)_2COOH$ | 2,6-di-F-Ph | $-(CH_2)_{1-3}$-2-indolyl | O |
| $NO_2$ | $-(CH_2)_2COOH$ | $-CH_2COO$-t-Bu | $-(CH_2)_{1-3}$-2-indolyl | O |
| $NO_2$ | $-(CH_2)_2COOH$ | $-CH_2COOEt$ | $-(CH_2)_{1-3}$-2-indolyl | O |
| H | H | Ph | $-(CH_2)_{1-3}$-2-indolyl | HH |
| H | H | Ph | $-(CH_2)_{1-3}$-3-indolyl | HH |
| H | H | p-Cl-Ph | $-(CH_2)_{1-3}$-2-indolyl | HH |
| H | H | p-Cl-Ph | $-(CH_2)_{1-3}$-3-indolyl | HH |
| H | H | o-F-Ph | $-(CH_2)_{1-3}$-2-indolyl | HH |
| H | H | o-F-Ph | $-(CH_2)_{1-3}$-3-indolyl | HH |
| H | H | 2,4-di-Cl-Ph | $-(CH_2)_{1-3}$-2-indolyl | HH |
| H | H | 2,4-di-Cl-Ph | $-(CH_2)_{1-3}$-3-indolyl | HH |

TABLE 2 (Cont'd)

| $X^1$ | $R^1$ | $R^2$ | $R^3$ | $X^7$ |
|---|---|---|---|---|
| H | H | 2,6-di-F-Ph | $-(CH_2)_{1-3}$-2-indolyl | HH |
| H | H | 2,6-di-F-Ph | $-(CH_2)_{1-3}$-3-indolyl | HH |
| H | H | $-CH_2COO$-t-Bu | $-(CH_2)_{1-3}$-2-indolyl | HH |
| H | H | $-CH_2COO$-t-Bu | $-(CH_2)_{1-3}$-3-indolyl | HH |
| H | H | $-CH_2COOEt$ | $-(CH_2)_{1-3}$-2-indolyl | HH |
| H | H | $-CH_2COOEt$ | $-(CH_2)_{1-3}$-3-indolyl | HH |
| H | $CH_3$ | Ph | $-(CH_2)_{1-3}$-2-indolyl | HH |
| H | $CH_3$ | p-Cl-Ph | $-(CH_2)_{1-3}$-2-indolyl | HH |
| H | $CH_3$ | o-F-Ph | $-(CH_2)_{1-3}$-2-indolyl | HH |
| H | $CH_3$ | 2,4-di-Cl-Ph | $-(CH_2)_{1-3}$-2-indolyl | HH |
| H | $CH_3$ | 2,6-di-F-Ph | $-(CH_2)_{1-3}$-2-indolyl | HH |
| H | $CH_3$ | $-CH_2COO$-t-Bu | $-(CH_2)_{1-3}$-2-indolyl | HH |
| H | $CH_3$ | $-CH_2COOEt$ | $-(CH_2)_{1-3}$-2-indolyl | HH |
| H | $-CH_2COOH$ | Ph | $-(CH_2)_{1-3}$-3-indolyl | HH |
| H | $-CH_2COOH$ | p-Cl-Ph | $-(CH_2)_{1-3}$-3-indolyl | HH |
| H | $-CH_2COOH$ | o-F-Ph | $-(CH_2)_{1-3}$-3-indolyl | HH |
| H | $-CH_2COOH$ | 2,4-di-Cl-Ph | $-(CH_2)_{1-3}$-3-indolyl | HH |
| H | $-CH_2COOH$ | 2,6-di-F-Ph | $-(CH_2)_{1-3}$-3-indolyl | HH |
| H | $-CH_2COOH$ | $-CH_2COO$-t-Bu | $-(CH_2)_{1-3}$-3-indolyl | HH |
| H | $-CH_2COOH$ | $-CH_2COOEt$ | $-(CH_2)_{1-3}$-3-indolyl | HH |

TABLE 3

Compounds of the Formula:

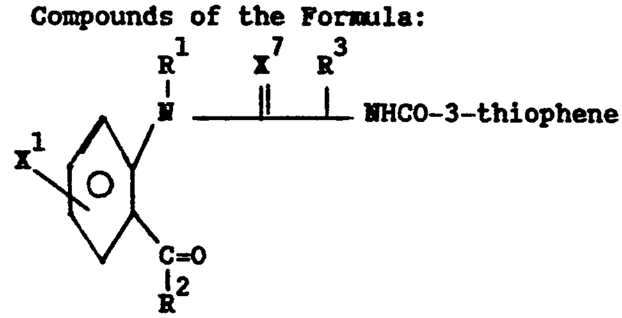

| $X^1$ | $R^1$ | $R^2$ | $R^3$ | $X^7$ |
|---|---|---|---|---|
| H | H | Ph | $-(CH_2)_{1-3}$-2-indolyl | O |
| H | H | Ph | $-(CH_2)_{1-3}$-3-indolyl | O |
| H | H | p-Cl-Ph | $-(CH_2)_{1-3}$-2-indolyl | O |
| H | H | p-Cl-Ph | $-(CH_2)_{1-3}$-3-indolyl | O |
| H | H | o-F-Ph | $-(CH_2)_{1-3}$-2-indolyl | O |
| H | H | o-F-Ph | $-(CH_2)_{1-3}$-3-indolyl | O |
| H | H | 2,4-di-Cl-Ph | $-(CH_2)_{1-3}$-2-indolyl | O |
| H | H | 2,4-di-Cl-Ph | $-(CH_2)_{1-3}$-3-indolyl | O |
| H | H | 2,6-di-F-Ph | $-(CH_2)_{1-3}$-2-indolyl | O |
| H | H | 2,6-di-F-Ph | $-(CH_2)_{1-3}$-3-indolyl | O |
| H | H | $-CH_2COO$-t-Bu | $-(CH_2)_{1-3}$-2-indolyl | O |
| H | H | $-CH_2COO$-t-Bu | $-(CH_2)_{1-3}$-3-indolyl | O |
| H | H | $-CH_2COOEt$ | $-(CH_2)_{1-3}$-2-indolyl | O |
| H | H | $-CH_2COOEt$ | $-(CH_2)_{1-3}$-3-indolyl | O |
| H | $CH_3$ | Ph | $-(CH_2)_{1-3}$-2-indolyl | O |
| H | $CH_3$ | p-Cl-Ph | $-(CH_2)_{1-3}$-2-indolyl | O |
| H | $CH_3$ | o-F-Ph | $-(CH_2)_{1-3}$-2-indolyl | O |
| H | $CH_3$ | 2,4-di-Cl-Ph | $-(CH_2)_{1-3}$-2-indolyl | O |
| H | $CH_3$ | 2,6-di-F-Ph | $-(CH_2)_{1-3}$-2-indolyl | O |
| H | $CH_3$ | $-CH_2COO$-t-Bu | $-(CH_2)_{1-3}$-2-indolyl | O |

TABLE 3 (Cont'd)

| $X^1$ | $R^1$ | $R^2$ | $R^3$ | $X^7$ |
|---|---|---|---|---|
| H | $CH_3$ | $-CH_2COOEt$ | $-(CH_2)_{1-3}$-2-indolyl | O |
| H | $-CH_2COOH$ | Ph | $-(CH_2)_{1-3}$-3-indolyl | O |
| H | $-CH_2COOH$ | p-Cl-Ph | $-(CH_2)_{1-3}$-3-indolyl | O |
| H | $-CH_2COOH$ | o-F-Ph | $-(CH_2)_{1-3}$-3-indolyl | O |
| H | $-CH_2COOH$ | 2,4-di-Cl-Ph | $-(CH_2)_{1-3}$-3-indolyl | O |
| H | $-CH_2COOH$ | 2,6-di-F-Ph | $-(CH_2)_{1-3}$-3-indolyl | O |
| H | $-CH_2COOH$ | $-CH_2COO$-t-Bu | $-(CH_2)_{1-3}$-3-indolyl | O |
| H | $-CH_2COOH$ | $-CH_2COOEt$ | $-(CH_2)_{1-3}$-3-indolyl | O |
| Cl | H | Ph | $-(CH_2)_{1-3}$-2-indolyl | O |
| Cl | H | Ph | $-(CH_2)_{1-3}$-3-indolyl | O |
| Cl | H | p-Cl-Ph | $-(CH_2)_{1-3}$-2-indolyl | O |
| Cl | H | p-Cl-Ph | $-(CH_2)_{1-3}$-3-indolyl | O |
| Cl | H | o-F-Ph | $-(CH_2)_{1-3}$-2-indolyl | O |
| Cl | H | o-F-Ph | $-(CH_2)_{1-3}$-3-indolyl | O |
| Cl | H | 2,4-di-Cl-Ph | $-(CH_2)_{1-3}$-2-indolyl | O |
| Cl | H | 2,4-di-Cl-Ph | $-(CH_2)_{1-3}$-3-indolyl | O |
| Cl | H | 2,6-di-F-Ph | $-(CH_2)_{1-3}$-2-indolyl | O |
| Cl | H | 2,6-di-F-Ph | $-(CH_2)_{1-3}$-3-indolyl | O |
| Cl | H | $-CH_2COO$-t-Bu | $-(CH_2)_{1-3}$-2-indolyl | O |
| Cl | H | $-CH_2COO$-t-Bu | $-(CH_2)_{1-3}$-3-indolyl | O |
| Cl | H | $-CH_2COOEt$ | $-(CH_2)_{1-3}$-2-indolyl | O |
| Cl | H | $-CH_2COOEt$ | $-(CH_2)_{1-3}$-3-indolyl | O |
| Cl | $CH_3$ | Ph | $-(CH_2)_{1-3}$-2-indolyl | O |
| Cl | $CH_3$ | p-Cl-Ph | $-(CH_2)_{1-3}$-2-indolyl | O |
| Cl | $CH_3$ | o-F-Ph | $-(CH_2)_{1-3}$-2-indolyl | O |
| Cl | $CH_3$ | 2,4-di-Cl-Ph | $-(CH_2)_{1-3}$-2-indolyl | O |
| Cl | $CH_3$ | 2,6-di-F-Ph | $-(CH_2)_{1-3}$-2-indolyl | O |
| Cl | $CH_3$ | $-CH_2COO$-t-Bu | $-(CH_2)_{1-3}$-2-indolyl | O |

0 166 355

TABLE 3 (Cont'd)

| $X^1$ | $R^1$ | $R^2$ | $R^3$ | $X^7$ |
|---|---|---|---|---|
| Cl | $CH_3$ | $-CH_2COOEt$ | $-(CH_2)_{1-3}$-2-indolyl | O |
| Cl | $-CH_2COOH$ | Ph | $-(CH_2)_{1-3}$-3-indolyl | O |
| Cl | $-CH_2COOH$ | p-Cl-Ph | $-(CH_2)_{1-3}$-3-indolyl | O |
| Cl | $-CH_2COOH$ | o-F-Ph | $-(CH_2)_{1-3}$-3-indolyl | O |
| Cl | $-CH_2COOH$ | 2,4-di-Cl-Ph | $-(CH_2)_{1-3}$-3-indolyl | O |
| Cl | $-CH_2COOH$ | 2,6-di-F-Ph | $-(CH_2)_{1-3}$-3-indolyl | O |
| Cl | $-CH_2COOH$ | $-CH_2COO$-t-Bu | $-(CH_2)_{1-3}$-3-indolyl | O |
| Cl | $-CH_2COOH$ | $-CH_2COOEt$ | $-(CH_2)_{1-3}$-3-indolyl | O |
| F | H | Ph | $-(CH_2)_{1-3}$-2-indolyl | O |
| F | H | Ph | $-(CH_2)_{1-3}$-3-indolyl | O |
| F | H | p-Cl-Ph | $-(CH_2)_{1-3}$-2-indolyl | O |
| F | H | p-Cl-Ph | $-(CH_2)_{1-3}$-3-indolyl | O |
| F | H | o-F-Ph | $-(CH_2)_{1-3}$-2-indolyl | O |
| F | H | o-F-Ph | $-(CH_2)_{1-3}$-3-indolyl | O |
| F | H | 2,4-di-Cl-Ph | $-(CH_2)_{1-3}$-2-indolyl | O |
| F | H | 2,4-di-Cl-Ph | $-(CH_2)_{1-3}$-3-indolyl | O |
| F | H | 2,6-di-F-Ph | $-(CH_2)_{1-3}$-2-indolyl | O |
| F | H | 2,6-di-F-Ph | $-(CH_2)_{1-3}$-3-indolyl | O |
| F | H | $-CH_2COO$-t-Bu | $-(CH_2)_{1-3}$-2-indolyl | O |
| F | H | $-CH_2COO$-t-Bu | $-(CH_2)_{1-3}$-3-indolyl | O |
| F | H | $-CH_2COOEt$ | $-(CH_2)_{1-3}$-2-indolyl | O |
| F | H | $-CH_2COOEt$ | $-(CH_2)_{1-3}$-3-indolyl | O |
| F | $CH_3$ | Ph | $-(CH_2)_{1-3}$-2-indolyl | O |
| F | $CH_3$ | p-Cl-Ph | $-(CH_2)_{1-3}$-2-indolyl | O |
| F | $CH_3$ | o-F-Ph | $-(CH_2)_{1-3}$-2-indolyl | O |
| F | $CH_3$ | 2,4-di-Cl-Ph | $-(CH_2)_{1-3}$-2-indolyl | O |
| F | $CH_3$ | 2,6-di-F-Ph | $-(CH_2)_{1-3}$-2-indolyl | O |
| F | $CH_3$ | $-CH_2COO$-t-Bu | $-(CH_2)_{1-3}$-2-indolyl | O |

TABLE 3 (Cont'd)

| $X^1$ | $R^1$ | $R^2$ | $R^3$ | $X^7$ |
|---|---|---|---|---|
| F | $CH_3$ | $-CH_2COOEt$ | $-(CH_2)_{1-3}$-2-indolyl | O |
| F | $-CH_2COOH$ | Ph | $-(CH_2)_{1-3}$-3-indolyl | O |
| F | $-CH_2COOH$ | p-Cl-Ph | $-(CH_2)_{1-3}$-3-indolyl | O |
| F | $-CH_2COOH$ | o-F-Ph | $-(CH_2)_{1-3}$-3-indolyl | O |
| F | $-CH_2COOH$ | 2,4-di-Cl-Ph | $-(CH_2)_{1-3}$-3-indolyl | O |
| F | $-CH_2COOH$ | 2,6-di-F-Ph | $-(CH_2)_{1-3}$-3-indolyl | O |
| F | $-CH_2COOH$ | $-CH_2COO$-t-Bu | $-(CH_2)_{1-3}$-3-indolyl | O |
| F | $-CH_2COOH$ | $-CH_2COOEt$ | $-(CH_2)_{1-3}$-3-indolyl | O |
| $CF_3$ | $CH_3$ | Ph | $-(CH_2)_{1-3}$-2-indolyl | O |
| $CF_3$ | $CH_3$ | p-Cl-Ph | $-(CH_2)_{1-3}$-2-indolyl | O |
| $CF_3$ | $CH_3$ | o-F-Ph | $-(CH_2)_{1-3}$-2-indolyl | O |
| $CF_3$ | $CH_3$ | 2,4-di-Cl-Ph | $-(CH_2)_{1-3}$-2-indolyl | O |
| $CF_3$ | $CH_3$ | 2,6-di-F-Ph | $-(CH_2)_{1-3}$-2-indolyl | O |
| $CF_3$ | $CH_3$ | $-CH_2COO$-t-Bu | $-(CH_2)_{1-3}$-2-indolyl | O |
| $CF_3$ | $CH_3$ | $-CH_2COOEt$ | $-(CH_2)_{1-3}$-2-indolyl | O |
| $CF_3$ | $CH_2CH_3$ | Ph | $-(CH_2)_{1-3}$-3-indolyl | O |
| $CF_3$ | $CH_2CH_3$ | p-Cl-Ph | $-(CH_2)_{1-3}$-3-indolyl | O |
| $CF_3$ | $CH_2CH_3$ | o-F-Ph | $-(CH_2)_{1-3}$-3-indolyl | O |
| $CF_3$ | $CH_2CH_3$ | 2,4-di-Cl-Ph | $-(CH_2)_{1-3}$-3-indolyl | O |
| $CF_3$ | $CH_2CH_3$ | 2,6-di-F-Ph | $-(CH_2)_{1-3}$-3-indolyl | O |
| $CF_3$ | $CH_2CH_3$ | $-CH_2COO$-t-Bu | $-(CH_2)_{1-3}$-3-indolyl | O |
| $CF_3$ | $CH_2CH_3$ | $-CH_2COOEt$ | $-(CH_2)_{1-3}$-3-indolyl | O |
| $CF_3$ | $-(CH_2)_2COOH$ | Ph | $-(CH_2)_{1-3}$-2-indolyl | O |
| $CF_3$ | $-(CH_2)_2COOH$ | p-Cl-Ph | $-(CH_2)_{1-3}$-2-indolyl | O |
| $CF_3$ | $-(CH_2)_2COOH$ | o-F-Ph | $-(CH_2)_{1-3}$-2-indolyl | O |
| $CF_3$ | $-(CH_2)_2COOH$ | 2,4-di-Cl-Ph | $-(CH_2)_{1-3}$-2-indolyl | O |
| $CF_3$ | $-(CH_2)_2COOH$ | 2,6-di-F-Ph | $-(CH_2)_{1-3}$-2-indolyl | O |
| $CF_3$ | $-(CH_2)_2COOH$ | $-CH_2COO$-t-Bu | $-(CH_2)_{1-3}$-2-indolyl | O |
| $CF_3$ | $-(CH_2)_2COOH$ | $-CH_2COOEt$ | $-(CH_2)_{1-3}$-2-indolyl | O |

TABLE 3 (Cont'd)

| $X^1$ | $R^1$ | $R^2$ | $R^3$ | $X^7$ |
|---|---|---|---|---|
| $NO_2$ | $CH_3$ | Ph | $-(CH_2)_{1-3}$-2-indolyl | O |
| $NO_2$ | $CH_3$ | p-Cl-Ph | $-(CH_2)_{1-3}$-2-indolyl | O |
| $NO_2$ | $CH_3$ | o-F-Ph | $-(CH_2)_{1-3}$-2-indolyl | O |
| $NO_2$ | $CH_3$ | 2,4-di-Cl-Ph | $-(CH_2)_{1-3}$-2-indolyl | O |
| $NO_2$ | $CH_3$ | 2,6-di-F-Ph | $-(CH_2)_{1-3}$-2-indolyl | O |
| $NO_2$ | $CH_3$ | $-CH_2COO$-t-Bu | $-(CH_2)_{1-3}$-2-indolyl | O |
| $NO_2$ | $CH_3$ | $-CH_2COOEt$ | $-(CH_2)_{1-3}$-2-indolyl | O |
| $NO_2$ | $CH_2CH_3$ | Ph | $-(CH_2)_{1-3}$-3-indolyl | O |
| $NO_2$ | $CH_2CH_3$ | p-Cl-Ph | $-(CH_2)_{1-3}$-3-indolyl | O |
| $NO_2$ | $CH_2CH_3$ | o-F-Ph | $-(CH_2)_{1-3}$-3-indolyl | O |
| $NO_2$ | $CH_2CH_3$ | 2,4-di-Cl-Ph | $-(CH_2)_{1-3}$-3-indolyl | O |
| $NO_2$ | $CH_2CH_3$ | 2,6-di-F-Ph | $-(CH_2)_{1-3}$-3-indolyl | O |
| $NO_2$ | $CH_2CH_3$ | $-CH_2COO$-t-Bu | $-(CH_2)_{1-3}$-3-indolyl | O |
| $NO_2$ | $CH_2CH_3$ | $-CH_2COOEt$ | $-(CH_2)_{1-3}$-3-indolyl | O |
| $NO_2$ | $-(CH_2)_2COOH$ | Ph | $-(CH_2)_{1-3}$-2-indolyl | O |
| $NO_2$ | $-(CH_2)_2COOH$ | p-Cl-Ph | $-(CH_2)_{1-3}$-2-indolyl | O |
| $NO_2$ | $-(CH_2)_2COOH$ | o-F-Ph | $-(CH_2)_{1-3}$-2-indolyl | O |
| $NO_2$ | $-(CH_2)_2COOH$ | 2,4-di-Cl-Ph | $-(CH_2)_{1-3}$-2-indolyl | O |
| $NO_2$ | $-(CH_2)_2COOH$ | 2,6-di-F-Ph | $-(CH_2)_{1-3}$-2-indolyl | O |
| $NO_2$ | $-(CH_2)_2COOH$ | $-CH_2COO$-t-Bu | $-(CH_2)_{1-3}$-2-indolyl | O |
| $NO_2$ | $-(CH_2)_2COOH$ | $-CH_2COOEt$ | $-(CH_2)_{1-3}$-2-indolyl | O |
| H | H | Ph | $-(CH_2)_{1-3}$-2-indolyl | HH |
| H | H | Ph | $-(CH_2)_{1-3}$-3-indolyl | HH |
| H | H | p-Cl-Ph | $-(CH_2)_{1-3}$-2-indolyl | HH |
| H | H | p-Cl-Ph | $-(CH_2)_{1-3}$-3-indolyl | HH |
| H | H | o-F-Ph | $-(CH_2)_{1-3}$-2-indolyl | HH |
| H | H | o-F-Ph | $-(CH_2)_{1-3}$-3-indolyl | HH |
| H | H | 2,4-di-Cl-Ph | $-(CH_2)_{1-3}$-2-indolyl | HH |
| H | H | 2,4-di-Cl-Ph | $-(CH_2)_{1-3}$-3-indolyl | HH |

TABLE 3 (Cont'd)

| $X^1$ | $R^1$ | $R^2$ | $R^3$ | $X^7$ |
|---|---|---|---|---|
| H | H | 2,6-di-F-Ph | $-(CH_2)_{1-3}$-2-indolyl | HH |
| H | H | 2,6-di-F-Ph | $-(CH_2)_{1-3}$-3-indolyl | HH |
| H | H | $-CH_2COO$-t-Bu | $-(CH_2)_{1-3}$-2-indolyl | HH |
| H | H | $-CH_2COO$-t-Bu | $-(CH_2)_{1-3}$-3-indolyl | HH |
| H | H | $-CH_2COOEt$ | $-(CH_2)_{1-3}$-2-indolyl | HH |
| H | H | $-CH_2COOEt$ | $-(CH_2)_{1-3}$-3-indolyl | HH |
| H | $CH_3$ | Ph | $-(CH_2)_{1-3}$-2-indolyl | HH |
| H | $CH_3$ | p-Cl-Ph | $-(CH_2)_{1-3}$-2-indolyl | HH |
| H | $CH_3$ | o-F-Ph | $-(CH_2)_{1-3}$-2-indolyl | HH |
| H | $CH_3$ | 2,4-di-Cl-Ph | $-(CH_2)_{1-3}$-2-indolyl | HH |
| H | $CH_3$ | 2,6-di-F-Ph | $-(CH_2)_{1-3}$-2-indolyl | HH |
| H | $CH_3$ | $-CH_2COO$-t-Bu | $-(CH_2)_{1-3}$-2-indolyl | HH |
| H | $CH_3$ | $-CH_2COOEt$ | $-(CH_2)_{1-3}$-2-indolyl | HH |
| H | $-CH_2COOH$ | Ph | $-(CH_2)_{1-3}$-3-indolyl | HH |
| H | $-CH_2COOH$ | p-Cl-Ph | $-(CH_2)_{1-3}$-3-indolyl | HH |
| H | $-CH_2COOH$ | o-F-Ph | $-(CH_2)_{1-3}$-3-indolyl | HH |
| H | $-CH_2COOH$ | 2,4-di-Cl-Ph | $-(CH_2)_{1-3}$-3-indolyl | HH |
| H | $-CH_2COOH$ | 2,6-di-F-Ph | $-(CH_2)_{1-3}$-3-indolyl | HH |
| H | $-CH_2COOH$ | $-CH_2COO$-t-Bu | $-(CH_2)_{1-3}$-3-indolyl | HH |
| H | $-CH_2COOH$ | $-CH_2COOEt$ | $-(CH_2)_{1-3}$-3-indolyl | HH |

The invention is further defined by reference to the following preparations and examples, which are intended to be illustrative and not limiting.

All temperatures are in degrees Celsius.

## EXAMPLE 1

2-N-($N^{\alpha}$-Boc-D-tryptophanyl)amino-5-chlorobenzophenone

2-Amino-5-chlorobenzophenone (7.65 g, 33 mmole), Boc-D-tryptophan (9.12 g, 30 mmole) and dicyclohexylcarbodiimide (DCC) (33 ml of a 1M solution in methylene chloride ($CH_2Cl_2$), 33 mmole) were combined in 45 ml of dry tetrahydrofuran (THF) stirred in an ice bath. The mixture was allowed to warm to room temperature and stirred overnight. The solids were removed by filtration and the filtrate evaporated *in vacuo*. The residue was chromatographed on 9" (23 cm) silica gel (230-400 mesh) in a 55 mm diameter column using 5% (v/v) diethylether in methylene chloride.

The product fractions were combined and evaporated *in vacuo*. The residue was crystallized from diethyl ether and the resulting solid dried *in vacuo* at 67° for 20 hours: (m.p. 90-92°, foam resolidifies; 150-152°).

The compound showed a single component by thin layer chromatography (TLC) ($R_f$= 0.24, silica gel plate eluted with 5% (v/v) diethyl ether in methylene chloride). The NMR spectrum was consistent with the title structure and verified the presence of $Et_2O$. The compound was 99.2% pure by HPLC. The mass spectrum showed a molecular ion at m/e=517.

Anal. Calc'd for $C_{29}H_{18}ClN_3O_4 \cdot C_4H_{10}O$:
C, 66.93; H, 6.47; N, 7.08; Cl, 5.99.
Found: C, 67.22; H, 6.54; N, 7.13; Cl, 6.11.

EXAMPLE 2

2-N-(N-α-Boc-L-tryptophanyl)amino-5-chlorobenzo phenone

The procedure of Example 1 was carried out using 2-amino-5-chlorobenzophenone (5.08 gm, 22.0 mmole), Boc-L-tryptophan (6.09 gm, 20 mmole) and DCC (22 ml of 1M solution in $CH_2Cl_2$, 22 mmole) in THF (30 ml). After filtration, evaporation *in vacuo* and chromatography (silica gel, 5% (v/v) $Et_2O$ in $CH_2Cl_2$), the title compound was obtained from $Et_2O$/hexane (1:2) as a white solid which was dried *in vacuo* at 67°C: (m.p. 152-4°C).

TLC: Single spot, $R_f$=0.24, silica gel, 5% (v/v) $Et_2O$ in $CH_2Cl_2$.

NMR: Spectrum consistent with title structure. $Et_2O$ present.

HPLC: 98.6% pure.

Mass Spec: Mol.ion at m/e = 517.

Anal. Calc'd for $C_{29}H_{28}ClN_3O_4 \cdot 0.5C_4H_{10}O$:
C, 67.08; H, 5.99; N, 7.57; Cl, 6.39.
Found: C, 67.33; H, 6.06; N, 7.69; Cl, 6.63.

EXAMPLE 3

2-N-($N^{\alpha}$-Boc-L-tryptophanyl)amino-2'-fluorobenzo phenone

The procedure of Example 1 was carried out using 2-amino-2'-fluorobenzophenone (5.0 gm, 23.2 mmole), Boc-L-tryptophan (7.07 gm, 23.2 mmole) and DCC (23.2 ml of 1M solution in $CH_2Cl_2$, 23.2

mmole) in THF (35 ml). After filtration, evaporation *in vacuo*, and chromatography (silica gel, 4% (v/v) $Et_2O$ in $CH_2Cl_2$), the title compound was obtained from $Et_2O$ as a white solid, which was dried *in vacuo* at 40°C: (m.p. 64-7°C).

TLC: Single spot, $R_f$=0.37, silica gel, 6% (v/v) $Et_2O$ in $CH_2Cl_2$.

NMR: Spectrum consistent with title structure, $Et_2O$ present.

HPLC: 99.5% pure.

Mass Spec: Mol.ion at m/e = 501.

Anal. Calc'd for $C_{29}H_{28}FN_3O_4 \cdot C_4H_{10}O$:

C, 68.85; H, 6.65; N, 7.30;

Found: C, 69.12; H, 6.65; N, 7.36.

EXAMPLE 4

2-N-($N^{\alpha}$-Boc-D-tryptophanyl)amino-2'-fluorobenzophenone

The procedure of Example 3 was carried out substituting Boc-D-tryptophan for Boc-L-tryptophan. Work-up and crystallization from $Et_2O$ gave the title compound as a white solid which was dried *in vacuo* at 40°C (m.p. 64-7°C).

TLC: Single spot, $R_f$=0.36, silica gel, 6% (v/v) $Et_2O$ in $CH_2Cl_2$.

NMR: Spectrum consistent with title structure, $Et_2O$ present.

HPLC: 99.5% pure.

Mass Spec: Mol.Ion at m/e = 501.

Anal. Calc'd for $C_{29}H_{28}FN_3O_4 \cdot C_4H_{10}O$:

C, 68.85; H, 6.65; N, 7.30;

Found: C, 69.25; H, 6.75; N, 7.30.

EXAMPLE 5

2-N-($N^{\alpha}$-Boc-L-tryptophanyl)aminobenzophenone

The procedure of Example 1 was carried out using 2-aminobenzophenone (5.0 gm, 25.3 mmole), Boc-L-tryptophan (7.70 gm, 25.3 mmole) and DCC (25.3 ml of 1M solution in $CH_2Cl_2$, 25.3 mmole) in THF (35 ml). After filtration, evaporation *in vacuo*, and chromatography (silica gel, 4% (v/v) $Et_2O$ in $CH_2Cl_2$), the title compound was obtained from $Et_2O$ as a white solid which was dried *in vacuo* at 40°C: (m.p. 74-80°C).

TLC: Single spot, $R_f$=0.34, silica gel, 6% $Et_2O$ in $CH_2Cl_2$.

NMR: Spectrum consistent with title structure, $Et_2O$ present.

HPLC: 99.7% pure.

Mass Spec: Mol.Ion at m/e = 483).

Anal. Calc'd for $C_{29}H_{29}N_3O_4 \cdot 0.8C_4H_{10}O$:
C, 71.24; H, 6.87; N, 7.74;

Found: C, 71.24; H, 7.04; N, 7.78.

EXAMPLE 6

2-N-($N^{\alpha}$-Boc-D-tryptophanyl)aminobenzophenone

The procedure of Example 5 was carried out substituting Boc-D-tryptophan for Boc-L-tryptophan. Work-up and crystallization from $Et_2O$ gave the title compound as a white solid, which was dried *in vacuo* at 40°C: (m.p. 74-80°C).

TLC: Single spot, $R_f$=0.34, silica gel, 6% (v/v) $Et_2O$ in $CH_2Cl_2$.

NMR: Spectrum consistent with title structure, $Et_2O$ present.

HPLC: 99.8% pure.

Mass Spec: Mol.Ion at m/e = 483.

Anal. Calc'd for $C_{29}H_{29}N_3O_4 \cdot 0.8C_4H_{10}O$:
C, 71.24; H, 6.87; N, 7.74;
Found: C, 71.34; H, 6.93; N, 7.59.

EXAMPLE 7

2-N-($N^{\alpha}$-Boc-DL-1-naphthylalanyl)aminobenzophenone

The procedure of Example 1 was carried out using 2-amino-5-chlorobenzophenone (845 mg, 3.65 mmole), Boc-DL-α-naphthylalanine (1.15 gm, 3.65 mmole), and DCC (3.65 ml of 1M solution in $CH_2Cl_2$, 3.65 mmole) in THF (5 ml). Filtration, evaporation *in vacuo*, and chromatography (silica gel, 1% (v/v)$Et_2O$ 1N $CH_2Cl_2$) gave the title compound from $Et_2O$ as a white solid which was dried *in vacuo* at 80°C: (m.p. 142-4°C).

TLC: Single spot, $R_f$=0.42, silica gel, 3% $Et_2O$ in $CH_2Cl_2$.

NMR: Spectrum consistent with title structure.

HPLC: 100% pure.

Anal. Calc'd for $C_{31}H_{29}ClN_2O_4$:
C, 70.38; H, 5.53; N, 5.30; Cl, 6.70;
Found: C, 70.55; H, 5.58; N, 5.13; Cl, 6.80.

EXAMPLE 8

2-2N-($N^{\alpha}$-Boc-DL-2-naphthylalanyl)aminobenzophenone

The procedure of Example 7 was carried out substituting BOC-DL-ß-naphthylalanine in place of BOC-DL-α-naphthylalanine. Work-up and crystallization from $Et_2O$ gave the title compound as a fluffy white solid which was dried *in vacuo* at 80°C: (m.p. 151-2°).

TLC: Single spot, $R_f$=0.40, silica gel, 3% (v/v) $Et_2O$ in $CH_2Cl_2$.
NMR: Spectrum consistent with title structure.
HPLC: 100% pure.
Anal. Calc'd for $C_{31}H_{29}N_2ClO_4$:
C, 70.38; H, 5.53; N, 5.30; Cl, 6.70;
Found: C, 70.55; H, 5.52; N, 5.35; Cl, 6.68.

## EXAMPLE 9

### 2-N-[[2(RS)-2-Bocamino-2-(3-thienyl)]acetyl]amino-2'-fluorobenzophenone

The procuedure of Example 1 was carried out using 2-amino-2'-fluorobenzophenone (1.59 gm, 7.40 mmole), DL-Bocamino-3-thienylacetic acid (2.0 gm, 7.77 mmole) and DCC (7.77 ml of 1.0 M solution in $CH_2Cl_2$, 7.77 mmole) in $CH_2Cl_2$ (15 ml). After filtration, evaporation *in vacuo*, and chromatography (silica gel, 3% (v/v) $Et_2O$ in $CH_2Cl_2$), the title compound was obtained from $Et_2O$ as a white solid which was dried *in vacuo* at 78°C: (m.p. 155-6°C).

TLC: Single spot, $R_f$=0.49, silica gel, 5% $Et_2O$ in $CH_2Cl_2$.
NMR: Spectrum consistent with title structure.
HPLC: 99.8% pure.
Anal. Calc'd for $C_{24}H_{23}FN_2O_4S$:
C, 63.42; H, 5.10; N, 6.16; F, 4.18;
Found: C, 63.55; H, 5.29; N, 6.50; F, 4.22.

## EXAMPLE 10

### 2-N-[[2(RS)-2-(3-thienoylamino)-2-(3-thienyl)]acetyl]-amino-2'-fluorobenzophenone

2-N-[[2(RS)-2-Bocamino-2-(2-thienyl)]acetyl] amino-2'-fluorobenzophenone (1.5 gm, 3.30 mmole) was

dissolved in EtOAc (20 ml). The solution was cooled to 0°C, saturated with HCl gas for 20 minutes, stirred 10 minutes and evaporated to dryness *in vacuo* to give a white solid. A portion of the white solid (650 mg, 1.66 mmole) was suspended in $CH_2Cl_2$ (20 ml) and treated wtih thiophene-3-carbonyl chloride (243 mg, 1.66 mmole) and $Et_3N$ (0.48 ml, 3.45 mmole). After 20 minutes at room temperature, the mixture was washed with 10% $NaHCO_3$ (aq.), $H_2O$, and brine, dried over $MgSO_4$, filtered and evaporated to dryness *in vacuo*. After chromatography (silica gel, 3% (v/v) $Et_2O$ in $CH_2Cl_2$) the title compound was obtained from $Et_2O$ as fine white needles which were dried *in vacuo* at 65°C (m.p. 156-8°C).

TLC: Single spot, $R_f$=0.39, silica gel, 4% $Et_2O$ in $CH_2Cl_2$.

NMR: Spectrum consistent with the title structure.

HPLC: 99.4% pure.

Anal. Calc'd for $C_{24}H_{17}FN_2O_3S_2$:
C, 62.05; H, 3.69; N, 6.03;

Found: C, 61.87; H, 3.57; N, 5.93.

## EXAMPLE 11

**2-N-($N^{\alpha}$-Boc-DL-ß-(2-thienyl)alanyl)amino-2'-fluorobenzophenone**

The procedure of Example 1 was carried out using 2-amino-2'-fluorobenzophenone (1.26 gm, 5.86 mmole), BOC-DL-ß-(2-thienyl)alanine (1.75 gm, 6.45 mmole) and DCC (6.45 ml of 1.0*M* solution in $CH_2Cl_2$, 6.45 mmole) in $CH_2Cl_2$ (25 ml). After filtration, evaporation *in vacuo* and chromatography (1% (v/v) $Et_2O$ in $CH_2Cl_2$), the title compound

was obtained from $Et_2O$/hexane as a white solid which was dried in vacuo at 65°C: (m.p. 88-93°C, cloudy; 120°C, clear).

TLC: Single spot, $R_f$=0.45, silica gel, 4% $Et_2O$ in $CH_2Cl_2$.

NMR: Spectrum consistent with title structure.

HPLC: 99.7% pure.

Anal. Calc'd for $C_{25}H_{25}FN_2O_4S$:
C, 64.08; H, 5.38; N, 5.98;

Found: C, 64.25; H, 5.50; N, 6.09.

EXAMPLE 12

2-N-($N^{\alpha}$-3-Thienoyl-DL-ß-(2-thienyl)alanyl)amino 2'-fluorobenzophenone

2-N-($N^{\alpha}$-Boc-DL-ß-(2-thienyl)alanyl)amino-2'-fluorobenzophenone (1.0 gm, 2.13 mmol) was dissolved in EtOAc (20 ml). The solution was cooled to 0°C, saturated wth HCl(g) for 20 minutes, stirred 10 minutes, and evaporated to dryness in vacuo to give a white foam. A portion of the foam (0.46 gm, 1.14 mmole) was suspended in $CH_2Cl_2$ (15 ml) and treated with thiophene-3-carbonyl chloride (166 mg, 1.14 mmol) and $Et_3N$ (0.33 m, 2.37 mmol). After stirring 20 minutes at room temperature, the mixture was washed with 10% $NaHCO_3$ (aq.), $H_2O$, and brine, dried over $MgSO_4$, filtered, and evaporated to dryness in vacuo. The title compound was obtained from $Et_2O$ as colorless crystals which were dried in vacuo at 65°C: (m.p. 152-5°C).

TLC: Single spot, $R_f$=0.38, silica gel, 5% (v/v) $Et_2O$ in $CH_2Cl_2$.

NMR: Spectrum consistent with title structure.

HPLC: 99.5% pure.

Anal. Calc'd for $C_{25}H_{19}FN_2O_3S_2$:
C, 62.74; H, 4.00; N, 5.85;
Found: C, 62.63; H, 4.01; N, 6.04.

EXAMPLE 13

2-N-[3-(3'indolyl)propanoyl]amino-5-chlorobenzophenone

The procedure of Example 1 was carried out using 2-amino-5-chlorobenzophenone (1.27 gm, 5.29 mmole), 3-indole propionic acid (1.0 gm, 5.29 mmole) and DCC (5.29 ml of 1M solution in $CH_2Cl_2$, 5.29 mmole) in THF (20 ml). After filtration, evaporation *in vacuo* and chromatography (silica gel, $CH_2Cl_2$), the title compound was obtained from ether/hexane as a light yellow foam. The foam was dried *in vacuo* at 25°C: (m.p. 53-61°C (shrink), 61-67°C (melt)).

TLC: Single spot, $R_f$ = 0.38, silica gel, 3% (v/v) $Et_2O$ in $CH_2Cl_2$.

NMR: Spectrum consistent with title structure.

HPLC: 99.8% pure.

Mass Spectrum: Mol. ion at m/e = 402.

Anal. Calc'd for $C_{24}H_{19}N_2O_2Cl$:
C, 71.55; H, 4.75; N, 6.95; Cl, 8.80.
Found: C, 71.34; H, 4.79; N, 6.81; Cl, 8.94.

EXAMPLE 14

2-N-(L-Tryptophanyl)aminobenzophenone hydrochloride

2-N-($N^{\alpha}$-Boc-L-tryptophanyl)aminobenzophenone (4.8 gm, 8.84 mmol) was dissolved in EtOAc (100 ml). The solution was cooled to 0°C, saturated with HCl (g) for 20 minutes, stirred 10 minutes and evaporated to dryness *in vacuo* to give, A, 2-N-(L-tryptophanyl)aminobenzophenone hydrochloride as a light yellow solid which was dried at 25° *in vacuo*.

A was used to prepare the compounds of Examples 15 thru 18.

EXAMPLE 15

2-N-($N^{\alpha}$-p-Chlorobenzoyl-L-tryptophanyl)aminobenzophenone

Compound A (Example 14) (420 mg, 1.0 mmole) was partitioned between $H_2O$ (5 ml) and $CH_2Cl_2$ (20 ml). 1N NaOH (2.0 ml) was added and the mixture cooled to 0°C. The mixture was treated with p-chlorobenzoylchloride (.127 ml, 1 mmole) followed by 1N NaOH (0.5 ml). After stirring 10 minutes, the mixture was extracted wih $CH_2Cl_2$ (2 x 25 ml). The combined organic extracts were washed with $H_2O$ and brine, dried over $MgSO_4$, filtered and evaporated to dryness *in vacuo*. After chromatography (silica gel, 7% (v/v) $Et_2O$ in $CH_2Cl_2$), the title compound was obtained from $Et_2O$ as a white foam which was dried *in vacuo* at 65°C (m.p. 95-105°C, shrink; 105-8°C, melt).

TLC: Single spot, $R_f$=0.68, silica gel, 20% $Et_2O$ in $CH_2Cl_2$.

NMR: Spectrum consistent with title structure.

HPLC: 98.5% pure.

Mass Spec: Mol.Ion at m/e = 521.

Anal. Calc'd for $C_{31}H_{24}ClN_3O_3$:

C, 71.33; H, 4.63; N, 8.05; Cl, 6.79;

Found: C, 71.16; H, 4.68; N, 8.03; Cl, 6.63.

EXAMPLE 16

2-N-($N^{\alpha}$-acetyl-L-tryptophanyl)aminobenzophenone

Compound A (Example 14) (420 mg, 1.0 mmole), was partitioned between $H_2O$ (5 ml) and $CH_2Cl_2$

(25 ml). 1N NaOH (2.0 ml) was added and the mixture cooled to 0°C. The mixture was treated with four equal portions of acetyl chloride (each = .071 ml, 1.0 mmole) and 1N NaOH (aq.) (each = 1 ml) added every 30 minutes. After stirring an additional 30 minutes, the mixture was extracted with $CH_2Cl_2$ (2 x 25 ml). The combined organic extracts were washed with $H_2O$ and brine, dried over $MgSO_4$, filtered and evaporated to dryness *in vacuo*. After chromatography (silica gel, 20% (v/v) $Et_2O$ in $CH_2Cl_2$), the title compound was obtained from $Et_2O$ as an off-white foam, which was dried *in vacuo* at 65°C: (mp 100-110°C, shrink; 110-114°C, melt).

TLC: Single spot, $R_f$=0.22, silica gel, 20% (v/v) $Et_2O$ in $CH_2Cl_2$.

NMR: Spectrum consistent with title structure, $Et_2O$ present.

HPLC: 99.8% pure.

Mass Spec: Mol.Ion= at m/e = 425.

Anal. Calc'd for $C_{26}H_{23}N_3O_3 \cdot 0.1C_4H_{10}O$:
C, 73.25; H, 5.59; N, 9.71;
Found: C, 72.87; H, 5.54; N, 10.00

## EXAMPLE 17

**2-N-($N^{\alpha}$-3-thienoyl-L-tryptophanyl)aminobenzophenone**

Compound A (Example 14) (300 mg, 0.714 mmole), thiophene-3-carboxylic acid (101 mg, 0.786 mmole), 1-ethyl-3-(3-dimethylaminopropyl)carbodiimide hydrochloride (EDC, 151 mg, 0.786 mmole) and 1-hydroxybenzotriazole hydrate (HBT, 106 mg, 0.786 mmole) were combined in freshly degassed DMF (2 ml) and stirred at room temperature. The pH of the solution was adjusted to 9.0-9.5 with $Et_3N$ and

stirring was continued for 24 hours. The mixture was evaporated *in vacuo*, treated with 10% $Na_2CO_3$ (aq.) and extracted with $CH_2Cl_2$ (3 x 20 ml). The combined extracts were washed with $H_2O$ (1 x 20 ml), 10% citric acid (1 x 20 ml), $H_2O$ (1 x 20 ml) and brine (1 x 20 ml), dried over $MgSO_4$, filtered and evaporated to dryness *in vacuo*. After chromatography (silica gel, $CH_2Cl_2$) the title compound was obtained from $CH_2Cl_2$ (after evaporation) as a light yellow solid. The solid was dried at 56°C: (m.p. 90-110°C, shrink, then melt).

TLC: Single spot, $R_f$=0.52, silica gel, 20% (v/v) $Et_2O$ in $CH_2Cl_2$.

NMR: Spectrum consistent with title structure.

HPLC: 99.2% pure.

Mass Spec: Mol.Ion=493.

Anal. Calc'd for $C_{29}H_{23}N_3O_3S$:

C, 70.57; H, 4.70; N, 8.51; S, 6.50;

Found: C, 70.52; H, 4.91; N, 8.36; S, 6.52.

EXAMPLE 18

2-N-($N^{\alpha}$-Boc-glycyl-L-tryptophanyl)aminobenzophenone

Compound A (Example 14) (1.75 g, 4.17 mmole), BOC-glycine (803 mg, 4.58 mmole), EDC (880 mg, 4.58 mmole), and HBT (619 mg, 4.58 mmole), were combined in freshly degassed DMF (13 ml) and stirred at 25°C. The pH of the solution was adjusted to 9.0-9.5 with $Et_3N$ and stirring was continued for 24 hours. The reaction was worked up as in Example 17. After chromatography (silica gel, 1:1 (v/v) EtOAc/hexane), the title compound was obtained from $Et_2O$ as a yellow foam which was dried *in vacuo* 40°C: (m.p. 77-95°C, shrink; 95-7°C, melt).

TLC: Single spot, $R_f$=0.22, silica gel, 25% (v/v) $Et_2O$ in $CH_2Cl_2$.
NMR: Spectrum consistent with title structure.
HPLC: 97% pure.
Anal. Calc'd for $C_{31}H_{32}N_4O_5 \cdot 0.2H_2O$:
C, 68.41; H, 6.00; N, 10.30;
Found: C, 68.30; H, 6.21; N, 10.29.

EXAMPLE 19

2-N-($N^{\alpha}$-Boc-L-methionyl-glycyl-L-tryptophanyl)aminobenzophenone

2-N-($N^{\alpha}$-Boc-glycyl-L-tryptophanyl)aminobenzophenone (1.46 gm, 2.7 mmole) was dissolved in EtOAc (20 ml). The solution was cooled to 0°C, saturated with HCl (g) for 20 minutes, stirred 10 minutes, and evaporated to dryness in vacuo. The solid residue was combined with Boc-L-methionine (617 mg, 2.47 mmole), EDC (475 mg, 2.47 mmole) and HBT (334 mg, 2.47 mmole) in freshly degassed DMF (15 ml) and stirred at 25°C. The pH of the solution was adjusted to 9.0-9.5 with $Et_3N$ and stirring was continued for 24 hours. The reaction was worked up as in Example 17. After chromatography (2 x, silica gel, 2% (v/v) MeOH in $CH_2Cl_2$), the title compound was isolated from $Et_2O$/hexane as a light yellow solid. The solid was dried *in vacuo* at 56°C (m.p. 95-108°C).
NMR: Spectrum consistent with title structure.
HPLC: 97.2% pure.
Mass spec: (FAB) M + H at m/e = 672.
Anal. Calc'd for $C_{36}H_{41}N_5O_6S$:
C, 64.36; H, 6.15; N, 10.43; S, 4.77;
Found: C, 64.29; H, 6.51; N, 10.32; S, 4.57.

EXAMPLE 20

2-N-($N^{\alpha}$-Benzyloxycarbonyl-L-tryosyl-L-methionyl-glycyl-L-tryptophanyl)aminobenzophenone

2-N-($N^{\alpha}$-Boc-L-methionyl-glycyl-L-tryptophanyl)aminobenzophenone (620 mg, 0.923 mmole) was dissolved in EtOAc (5 ml). The solution was cooled to 0°C, saturated with HCl (g) for 20 minutes, stirred 10 minutes, and evaporated to dryness *in vacuo*. The solid residue was added to a suspension of $N^{\alpha}$-CBZ-L-tyrosine (161 mg, 0.512 mmole), N-methyl morpholine (NMM, 0.056 ml, 0.512 mmole) and isobutylchloroformate (0.66 ml, 0.512 mmole) stirring in EtOAc (4 ml) at -5°C. A second portion of NMM (0.056 ml, 0.512 mmole) was added immediately and the reaction stirred 3 hours. The reaction was treated with $H_2O$ (5 ml) and extracted with EtOAc (3 x 5 ml). The combined organic layers were washed with 0.5M citric acid (2 x), $H_2O$ (2x), 1N $NaHCO_3$ (aq) (2x), $H_2O$ (2x), and brine, dried over $MgSO_4$, filtered and evaporated to dryness *in vacuo*. After chromatography (silica gel, EtOAc, then silica gel, 180/10/1/1(v/v/v/v) $CH_2Cl_2$/MeOH/$H_2O$/HoAc), the title compound was obtained from $Et_2O$ (after evaporation) as an off-white solid. The solid was dried *in vacuo* at 65°C: (m.p. 125-40°C).

TLC: Single spot, $R_f$=0.19, silica gel, 180/10/1/1 (v/v/v/v) of $CH_2Cl_2$/MeOH/$H_2O$/HoAc.

NMR: Spectrum consistent with title structure.

HPLC: 98.3% pure.

Mass spec: (FAB) M + H at m/e = 869.

Anal. Calc'd for: $C_{48}H_{48}N_6O_8S$:

C, 66.34; H, 5.57; N, 9.67;

Found: C, 66.47; H, 5.83; N, 9.53.

EXAMPLE 21

2-N-($N^{\alpha}$-3-thienoyl-L-tryptophanyl)amino-5-chlorobenzophenone

2-N-(N'-Boc-L-tryptophanyl)amino-5-chlorobenzophenone (4.0 gm, 7.72 mmol) was dissolved in EtOAc (50 ml). The solution was cooled to 0°C, saturated with HCl(g) for 20 minutes, stirred 10 minutes and evaporated to dryness in vacuo. The solid residue was suspended in $CH_2Cl_2$ (50 ml), cooled to 0°C, and treated with thiophene-3-carbonyl chloride (1.13 gm, 7.72 mmole). The pH was adjusted to 7.4-7.6 with $Et_3N$. After stirring 15 minutes, the organic solution was washed with 10% $NaHCO_3$(%) and brine, dried over $MgSO_4$, filtered, and evaporated to dryness in vacuo. After flash chromatography (silica gel, 5% $Et_2O$ in $CH_2Cl_2$) the title compound was obtained from $Et_2O/CH_2Cl_2$ as an off-white solid. The compound was dried in vacuo at 78°C (m.p. 110-4°C). TLC: Single spot, $R_f$=0.26, silica GF, 10% $Et_2O$ in $CH_2Cl_2$.

NMR: spectrum consistent with the title structure, $CH_2Cl_2$ present.

HPLC: 99.7% pure

Mass. Spec.: Mol Ion at m/e=527.

Anal. Calc'd for $C_{29}H_{22}N_3ClO_3S.0.43\ CH_2Cl_2$:

C, 62.61; H, 4.08; N, 7.44; Cl, 11.68.

Found: C, 62.34; H, 4.17; N, 7.36; Cl, 11.50.

EXAMPLE 22

2-N-[2(S)-[2-(3'α-2',3'-dihydroindolyl)methyl-2-(3-thienoylamino)]acetyl]amino-5-chlorobenzophenone (A)
and
2-N-[2(S)-[2-(3'ß-2',3'-dihydroindolyl)methyl-2-(3-thienoylamino)]acetyl]amino-5-chlorobenzophenone (B)

The compound of Example 21 (3.45 gm, 6.53 mmol) was dissolved in triflfuoroacetic acid (30 ml), treated with triethylsilane (7 ml, 43.9 mmol), and stirred at room temperature. After 30 minutes, the reaction was evaporated to dryness *in vacuo* and the residue was treated with $H_2O$, basified with sat'd $Na_2CO_2$ (aq.), and extracted with ETOAc (3X). The combined organic extracts were washed with $H_2O$ and brine, dried over $MgSO_4$, filtered and evaporated to dryness *in vacuo*. The resulting pair of diastereomers were separated by chromatography (silica gel, 30-50%, $Et_2O$ in $CH_2Cl_2$) and evaporated to dryness *in vacuo*.

A. The faster running component , compound A, (TLC-$R_f$=0.29, silica GF, 40% $Et_2O$ in $CH_2Cl_2$) was isolated from $Et_2O$ as a yellow foam. The compound was dried *in vacuo* at 69°C (m.p. 88-92°C, shrink, 105-8°C, melt).

TLC: single spot.

NMR: spectrum was consistent with the title structure.

HPLC: 96% pure.

Mass. Spec.: Mol. ion at m/e=529.

Anal. Calc'd for $C_{29}H_{24}ClN_3O_3S$:

C, 65.71; H, 4.56; N, 7.93; Cl, 6.69.

Found: C, 65.55; H, 4.64; N, 7.76; Cl, 6.46.

B. The slower running component, compound B, (TLC-$R_f$=0.19, silica GF, 40% $Et_2O$ in $CH_2Cl_2$) was isolated from $Et_2O$ as a yellow foam. The compound was dried *in vacuo* at 69°C (m.p. 97-110°C).

TLC: single spot.

NMR: spectrum was consistent with the title structure.

HPLC: 97.4% pure.

Mass spec: Mol. Ion at m/e=529.

Anal. Calc'd for $C_{29}H_{24}ClN_3O_3S$:
C, 65.71; H, 4.56; N, 7.93; Cl, 6.69.

Found: C, 65.88; H, 4.72; N, 7.86; Cl, 6.65.

## EXAMPLE 23

2-N-[2-(S)-[2-[3α'-1'-(Boc-L-leucyl)-2',3'-dihydro-indolyl]methyl-2-(3-thienoylamino)]acetyl]amino-5-chlorobenzophenone

Compound A of Example 22 (100 mg, 0.189 mmole), Boc-L-Leucine monohydrate (51.8 mg, 0.208 mmole), and 1-hydroxybenzotriazole hydrate (HBT, 28.0 mg, 0.208 mmol) were dissolved in $CH_2Cl_2$ (4 ml). The solution was cooled to 0°C and treated with DCC (0.208 ml of a 1M solution in $CH_2Cl_2$). The mixture was stirred at 25°C for 24 hours, then filtered and evaporated to dryness *in vacuo*. After chromatography (silica gel, 10% $Et_2O$ in $CH_2Cl_2$) the title compound was obtained from $Et_2O$ (after evaporation) as a lt. yellow solid. The compound was dried *in vacuo* at 78°C (m.p. 120-30°).

TLC: single spot, $R_f$=0.64, silica GF, 15% $Et_2O$ in $CH_2Cl_2$.

NMR: spectrum was consistent with the title structure.

HPLC: single stereoisomer (99.0%) whose absolute configuration is undetermined.

Mass spec.: (FAB) m/e=743 (M+H).

Anal. Calc'd for $C_{40}H_{43}N_4ClO_6S$:

C, 64.63; H, 5.83; N, 7.54; Cl, 4.77.

Found: C, 64.55; H, 6.05; N, 7.25; Cl, 4.75.

## EXAMPLE 24

2-N-[2(S)-[2-[3'ß-1'-(Boc-L-leucyl)-2',3'-dihydro-indolyl]methyl-2-(3-thienoylamino)]acetyl]amino-5-chlorobenzophenone

The procedure of Example 23 was carried out using the same reagents and quantitites except compound B of Example 22 was substituted for compound A of Example 22. The title compound was obtained from $Et_2O$ (after evaporation) as an off-white solid. The solid was dried *in vacuo* at 78°C (m.p. 123-32°C, foam).

TLC: single spot, $R_f$=0.51, silica GF, 15% $Et_2O$ in $CH_2Cl_2$.

NMR: spectrum was consistent with the title structure.

HPLC: compound was a single stereoisomer (98.5%) whose absolute configuration is undetermined.

Mass Spec.: (FAB) m/e=743 (M+H).

Anal. Calc'd for $C_{40}H_{43}N_4ClO_6S$:

C, 64.63; H, 5.83; N, 7.54; Cl, 4.77.

Found: C, 64.52; H, 5.68; N, 7.45; Cl, 4.80.

EXAMPLE 25

2-N-[2(S)-[2-[3'α-1'-(Boc-D-leucyl)-2',3'-dihydro-indolyl]methyl-2-(3-thienoylamino)]acetyl]amino-5-chlorobenzophenone

The procedure of Example 23 was carried out using the same reagents and quantities except Boc-D-Leucine monohydrate was substituted for Boc-L-Leucine monohydrate. The title compound was obtained from $Et_2O$ (after evaporation) as a white powder. The compound was dried *in vacuo* at 78°C (m.p. 117-23°C, foam).

TLC: single spot, $R_f$=0.50, silica GF, 15% $Et_2O$ in $CH_2Cl_2$.

NMR: spectrum was consistent with the title structure.

HPLC: compound was a single stereoisomer (99.2%) whose absolute configuration is undetermined.

Mass Spec. (FAB): m/e=743 (M+H).

Anal. Calc'd for $C_{40}H_{43}N_4ClO_6S$:
C, 64.63; H, 5.83; N, 7.54; Cl, 4.77.
Found: C, 64.92; H, 6.23; N, 7.53; Cl, 4.77.

EXAMPLE 26

2-N-[2(S)-[2-[3'β-1'-(Boc-D-leucyl)-2',3'-dihydro-indolyl]methyl-2-(3-thienoylamino)]acetyl]amino-5-chlorobenzophenone

The procedure of Example 23 was carried out using the same reagents and quantities except compound B of Example 22 and Boc-D-Leucine monohydrate were substituted for compound A of Example 22 and Boc-L-Leucine monohydrate, respectively. The title compound was obtained from

$Et_2O$ (after evaporation) as an off-white foam. The compound was dried in vacuo at 78°C: (m.p. 122-30°C, foam).

TLC: single spot, $R_f$=0.50, silica GF, 15% $Et_2O$ in $CH_2Cl_2$.

NMR: spectrum was consistent with the title structure.

HPLC: compound was a single stereoisomer (99.5%) whose absolute configuration is undetermined.

Mass Spec. (FAB): m/e=743 (M+H).

Anal. Calc'd for $C_{40}H_{43}N_4ClO_6S$:
C, 64.63; H, 5.83; N, 7.54; Cl, 4.77.

Found: C, 64.77; H, 6.03; N, 7.61; Cl, 4.89.

## EXAMPLE 27

2-N-[3-(3'-indolyl)propanoyl]amino-5-chlorobenzophenone

2-Amino-5-chlorobenzophenone (1.22 gm, 5.29 mmole), 3-indole propionic acid (1.0 gm, 5.29 mmole) and DCC (5.29 ml of 1M solution in $CH_2Cl_2$) were combined in THF (20 ml). The mixture was stirred 18 hours at room temperature. After filtration, evaporation in vacuo and chromatography (silica gel, $CH_2Cl_2$) the title compound was obtained from ether/hexane as a light yellow foam. The foam was dried in vacuo at 25°C: (m.p. 53-61°C (shrink), 61-7°C (melt)).

TLC: single spot, $R_f$=0.38, silica GF, 3% $Et_2O$ in $CH_2Cl_2$.

NMR: spectrum was consistent with the title structure.

HPLC: 99.8% pure.

Mass Spec.: Mol Ion at m/e=402.

Anal. Calc'd for $C_{24}H_{19}N_2O_2Cl$:
C, 71.55; H, 4.75; N, 6.95; Cl, 8.80.
Found: C, 71.34; H, 4.79; N, 6.81; Cl, 8.94.

EXAMPLE 28

1-($N^{\alpha}$-Boc-L-tryptophanylamino)-2-(N-phenylamino) benzene

N-Phenyl-N-(2-aminophenyl)amine (0.92 g, 5 mmole), Boc-L-tryptophan (1.52 g, 5 mmole), and dicyclohexylcarbodiimide (5 mL of a 1 M solution in $CH_2Cl_2$, 5 mmole) were combined in dry tetrahydrofuran (7 ml) and stirred at ambient temperature for 48 hours. The mixture was filtered and evaporated *in vacuo*, and the residue was chromatographed on silica gel (230-400 mesh, 9"(23 cm) column, 25 mm diameter, $CH_2Cl_2$ and 5% (v/v) $Et_2O/CH_2Cl_2$ elution). The product fractions were evaporated *in vacuo* and the residue crystallized from $Et_2O$/hexane. The solid product was dried *in vacuo* at 40°: (m.p. 95-100°).

Analysis Calc'd for $C_{28}H_{30}N_4O_3$:
C, 71.47, H, 6.43, N, 11.91.
Found: C, 71.52; H, 6.65; N, 11.95.
TLC: single component $R_f$=0.46, silica gel plate eluted with 15% (v/v) $Et_2O$/ $CH_2Cl_2$.
NMR: spectrum was consistent with the title structure.
Mass spec: Mol. Ion at m/e=470.
HPLC: 97.8% pure.

WHAT IS CLAIMED IS:

1. A compound of Formula I:

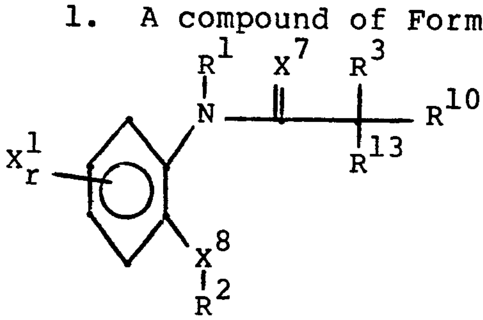

I

wherein

$R^1$ is H, $C_1$-$C_5$ linear or branched alkyl, loweralkenyl, $-(CH_2)_mCOOR^6$, $-(CH_2)_n$-cycloloweralkyl, or $-(CH_2)_mNR^4R^5$;

$R^2$ is H, loweralkyl, substituted or unsubstituted phenyl (wherein the substitutents may be 1 or 2 of halo, loweralkyl, loweralkoxy, carboxyl, carboxyloweralkyl, nitro, $-CF_3$, or hydroxy), $-(CH_2)_mSCH_3$, $-(CH_2)_mSOCH_3$, $-(CH_2)_mSO_2CH_3$, or $-(CH_2)_nCOOR^6$;

$R^3$ is $-(CH_2)_nR^7$, $-(CH_2)_nCH(OH)R^7$, $-(CH_2)_n-C(OH)(R^7_a)-R^7$

$-(CH_2)_nC(=O)R^7$, $-(CH_2)_nNH(CH_2)_qR^7$,

$-(CH_2)_mNHCH[(CH_2)_qR^7]COOR^6$, $-(CH_2)_mNHC(=O)(CH_2)_qR^7$,

or $-(CH_2)_2NHC(=O)CH(NHCOOR^{14})CH_2R^7$;

$R^4$ and $R^5$ are independently H, loweralkyl, or cycloloweralkyl;

$R^6$ is H, loweralkyl, cycloloweralkyl, substituted or unsubstituted phenyl, or substituted or unsubstituted phenylloweralkyl (wherein the substituents may be 1 or 2 of halo, loweralkyl, loweralkoxy, nitro, or $CF_3$);

$R^7$ and $R^7_a$ are independently α- or ß-naphthyl,

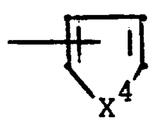 , 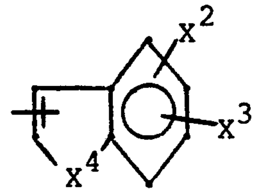 , 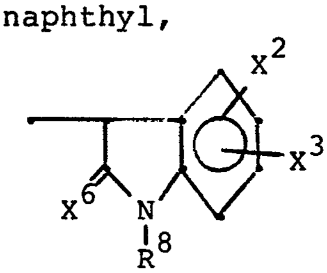

loweralkyl, substituted or unsubstituted phenyl (wherein the substituents may be 1 to 2 of halo, $-NO_2$, -OH, $-NR^4R^5$, loweralkyl, or loweralkoxy),

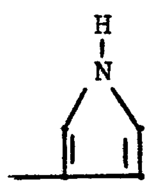 , 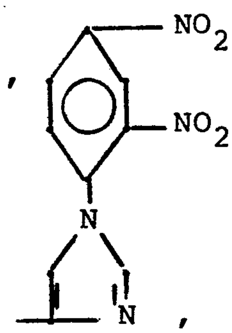 ,

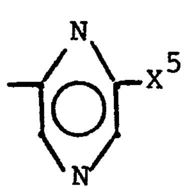 , or

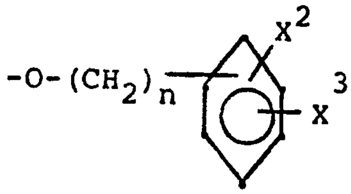

(with the proviso that q is not 1 in $-(CH_2)_nNH(CH_2)_q\ R^7$ and that q is not 0 in

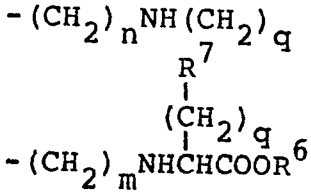

when $R^7$ is

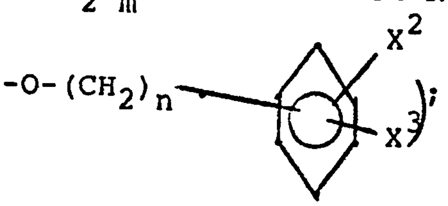

;

$R^8$ is H, loweralkyl, cycloloweralkyl,

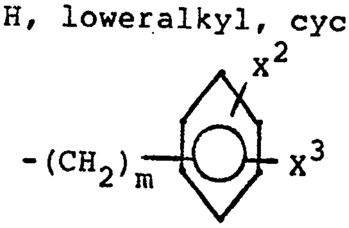

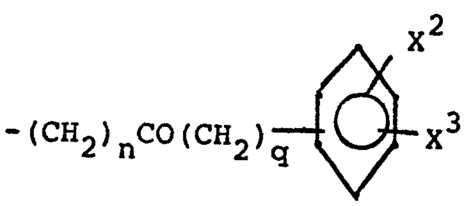

, or

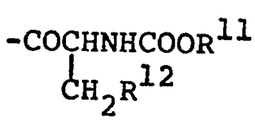

;

$R^{10}$ is H, -OH, or $-CH_3$;

$R^{11}$ and $R^{12}$ are independently loweralkyl or cycloloweralkyl;

$R^{13}$ is H, or $-NH\overset{\overset{O}{\|}}{C}R^{16}$;

$R^{14}$ is loweralkyl or phenylloweralkyl;

$R^{16}$ is H, loweralkyl, $-O(CH_2)_n$ 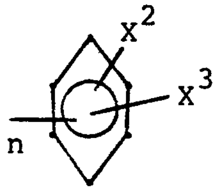 ,

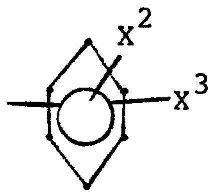 , 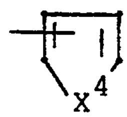 ,

$-(CH_2)_nNHCOOR^{11}$, $-(CH_2)_nNHCO\overset{H}{\underset{NHCOOR^{11}}{C}}(CH_2)_nX^4R^{12}$,

$OR^{11}$, or $-(CH_2)_m\overset{H}{N}R^{17}$;

$R^{17}$ is $A_1A_2A_3-$, $A_1A_3-$, or $A_1-$;

where

$A_1$ is H, Boc, Cbz, or acetyl;

$A_2$ is Tyr, Tyr-O-sulfate or phenyl;

$A_3$ is Met, nor-Leu, or nor-Val;

m is 1-4;

n is 0-4;

q is 0-4;

r is 1 or 2;

$X^1$ is H, $-NO_2$, $CF_3$ CN, OH, loweralkyl, halo, lower-alkylthio, loweralkoxy, $-(CH_2)_nCOOR^6$, or $-NR^4R^5$;

$X^2$ and $X^3$ are independently H, -OH, $-NO_2$, halo, lower-alkylthio, loweralkyl, or loweralkoxy;

$X^4$ is S, O, or $NR^8$;

$X^5$ is H, $CF_3$, CN, $COOR^6$, $NO_2$, or halo;

$X^6$ is O or HH;
$X^7$ is O, or HH;

$X^8$ is >C=O, $-\overset{|}{N}R^{10}$, or $-CH_2-$ ;

and salts and quaternary ammonium salts of the compounds of formula I.

2. A compound of claim 1 wherein in formula I

$R^1$ is H, loweralkyl, or $-(CH_2)_mCOOR^6$;
$R^2$ is substituted or unsubstituted phenyl (wherein the substitutents may be 1 or 2 of halo, loweralkyl, loweralkoxy, carboxyl, carboxyloweralkyl, nitro, $-CF_3$, or hydroxy), or $-(CH_2)_nCOOR^6$;

$R^3$ is $-(CH_2)_nR^7$;

$R^6$ is H, loweralkyl, cycloloweralkyl, substituted or unsubstituted phenyl, or substituted or unsubstituted phenylloweralkyl (wherein the substituents may be 1 or 2 of halo, loweralkyl, loweralkoxy, nitro, or $CF_3$);
$R^7$ is α- or ß-naphthyl,

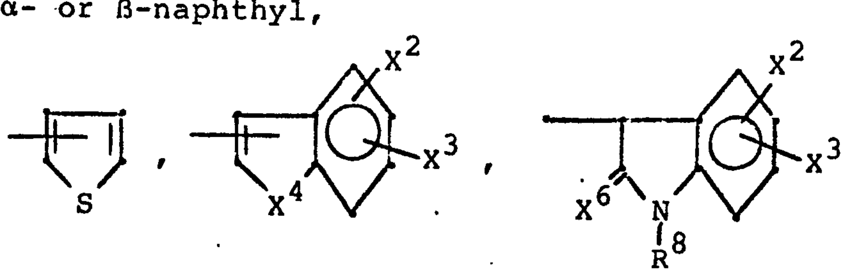

loweralkyl, substituted or unsubstituted phenyl (wherein the substituents may be 1 to 2

of halo, $-NO_2$, -OH, $-NR^4R^5$, loweralkyl, or loweralkoxy),

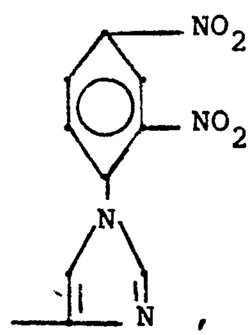

, or

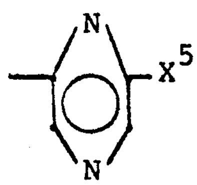

;

$R^8$ is H or loweralkyl;

$R^4$ and $R^5$ are independently hydrogen, loweralkyl or cycloloweralkyl;

$R^{10}$ is hydrogen;

$R^{13}$ is H, or $-NH\overset{O}{\overset{\|}{C}}R^{16}$;

$R^{16}$ is $-O(CH_2)_n$ 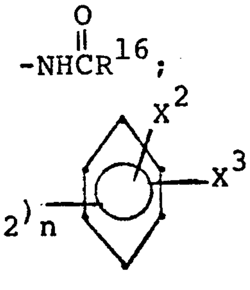 ,

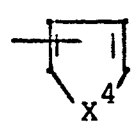 ,

$OR^{11}$, or $-(CH_2)_m\overset{H}{N}R^{17}$;

$R^{11}$ is loweralkyl;

$R^{17}$ is $A_1A_2A_3$-, $A_1A_3$-, or $A_1$-;

where

$A_1$ is H, Boc, Cbz, or acetyl;

$A_2$ is Tyr, Tyr-O-sulfate or phenyl;

$A_3$ is Met, nor-Leu, or nor-Val;

m is 1-4;

n is 0-4;

r is 1 or 2;

$X^1$ is H, $-NO_2$, $CF_3$ CN, or halo;

$X^2$ and $X^3$ are independently H, -OH, $-NO_2$, halo, loweralkyl, or loweralkoxy;

$X^4$ is S, O, or $NR^8$;

$X^5$ is H, $CF_3$, CN, $COOR^6$, or halo;

$X^6$ is O or HH;

$X^7$ is O, or HH;

$X^8$ is C=O;

and salts and quaternary ammonium salts of the compounds of formula I.

3. A compound of claim 1 or 2 having the formula I wherein

$R^1$ is H, methyl, ethyl, or $-(CH_2)_{1-2}COOR^6$;

$R^2$ is substituted or unsubstituted phenyl (wherein the substitutents may be 1 or 2 of halo, or carboxyl), or $-(CH_2)_{1-2}COOR^6$;

$R^3$ is $-(CH_2)_nR^7$;

$R^6$ is H or loweralkyl;

$R^7$ is α- or ß-naphthyl, loweralkyl, unsubstituted phenyl,

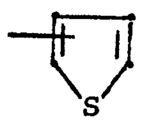 , 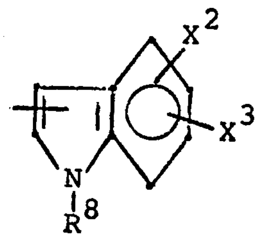 , 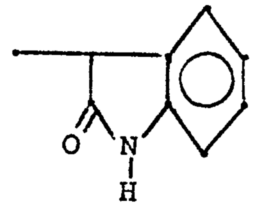 ,

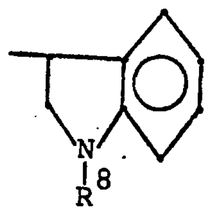 , 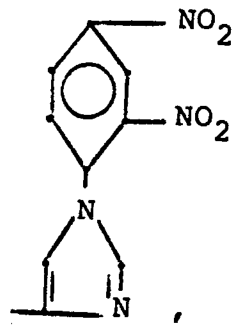 , or

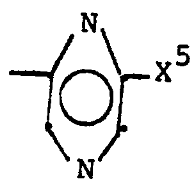 ;

$R^8$ is H, methyl, or ethyl;
$R^{10}$ is H;

$R^{13}$ is H, or $-NH\overset{O}{\overset{\|}{C}}R^{16}$;

$R^{16}$ is 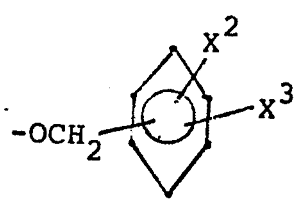 , 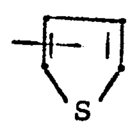 , or

$-OC(CH_3)_3$;

m is 1-4;
n is 0-4;

r is 1 or 2;
$X^1$ is H, $-NO_2$, $CF_3$ CN, or halo;
$X^2$ and $X^3$ are independently H, -OH, $-NO_2$, or halo;
$X^5$ is H or halo;
$X^7$ is O or HH;
$X^8$ is C=O;
and salts and quaternary ammonium salts of the compounds of formula I.

4. A compound according to one of the claims 1 - 3, having the formula I wherein
$R^1$ is H, methyl, ethyl, or $-(CH_2)_{1-2}COOH$;
$R^2$ is phenyl, o-fluorophenyl, p-chlorophenyl, o-carboxyphenyl, 2,6-difluorophenyl, $-CH_2COOEt$, or $-CH_2COO-t-Bu$;
$R^3$ is $-(CH_2)_nR^7$;
$R^7$ is α- or ß-naphthyl,

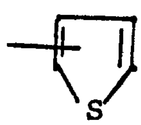 or 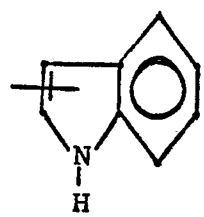 ;

$R^{10}$ is H;

$R^{13}$ is H, or $-NH\overset{O}{\overset{\|}{C}}R^{16}$;

$R^{16}$ is 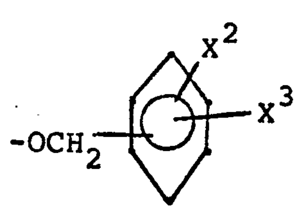 , 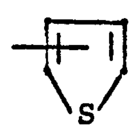 , or

$-OC(CH_3)_3$;

m is 1-4;
n is 0-4;
q is 0-4;
r is 1;
$X^1$ is H, 4-chloro, 4-fluoro, or 4-nitro;
$X^2$ and $X^3$ are independently H, -OH, fluoro, or bromo;
$X^7$ is O;
$X^8$ is C=O;
and salts and quaternary ammonium salts of the compounds of formula I.

5. A compound according to one of the claims 1 - 4 having the formula I, which is
2-N-($N^{\alpha}$-Boc-D-tryptophanyl)amino-5-chlorobenzophenone;
2-N-(N-α-Boc-L-tryptophanyl)amino-5-chlorobenzo phenone;
2-N-($N^{\alpha}$-Boc-L-tryptophanyl)amino-2'-fluorobenzo phenone;
2-N-($N^{\alpha}$-Boc-D-tryptophanyl)amino-2'-fluorobenzo phenone;
2-N-($N^{\alpha}$-Boc-L-tryptophanyl)aminobenzophenone;
2-N-($N^{\alpha}$-Boc-D-tryptophanyl)aminobenzophenone;
2-N-($N^{\alpha}$-Boc-DL-1-naphthylalanyl)aminobenzophenone;
2-2N-($N^{\alpha}$-Boc-DL-2-naphthylalanyl)aminobenzophenone;
2-N-[[2(RS)-2-Bocamino-2-(3-thienyl)]acetyl]amino-2'-fluorobenzophenone;

2-N-[[2(RS)-2-(3-thienoylamino)-2-(3-thienyl)]acetyl]-amino-2'-fluorobenzophenone;

2-N-($N^{\alpha}$-Boc-DL-ß-(2-thienyl)alanyl)amino-2'-fluorobenzophenone;

2-N-($N^{\alpha}$-3-Thienoyl-DL-ß-(2-thienyl)alanyl)amino 2'-fluorobenzophenone;

2-N-[3-(3'indolyl)propanoyl]amino-5-chlorobenzophenone;

2-N-($N^{\alpha}$-p-Chlorobenzoyl-L-tryptophanyl)aminobenzophenone;

2-N-($N^{\alpha}$-acetyl-L-tryptophanyl)aminobenzophenone;

2-N-($N^{\alpha}$-3-thienoyl-L-tryptophanyl)aminobenzophenone;

2-N-($N^{\alpha}$-Boc-glycyl-L-tryptophanyl)aminobenzophenone;

2-N-($N^{\alpha}$-Boc-L-methionyl-glycyl-L-tryptophanyl)aminobenzophenone;

2-N-($N^{\alpha}$-Benzyloxycarbonyl-L-tryosyl-L-methionyl-glycyl-L-tryptophanyl)aminobenzophenone;

2-N-($N^{\alpha}$-3-thienoyl-L-tryptophanyl)amino-5-chlorobenzophenone;

2-N-[2(S)-[2-(3'α-2',3'-dihydroindolyl)methyl-2-(3-thienoylamino)]acetyl]amino-5-chlorobenzophenone;

2-N-[2(S)-[2-(3'ß-2',3'-dihydroindolyl)methyl-2-(3-thienoylamino)]acetyl]amino-5-chlorobenzophenone;

2-N-[2-(S)-[2-[3α'-1'-(Boc-L-leucyl)-2',3'-dihydroindolyl]methyl-2-(3-thienoylamino)]acetyl]amino-5-chlorobenzophenone;

2-N-[2(S)-[2-[3'ß-1'-(Boc-L-leucyl)-2',3'-dihydroindolyl]methyl-2-(3-thienoylamino)]acetyl]amino-5-chlorobenzophenone;

2-N-[2(S)-[2-[3'α-1'-(Boc-D-leucyl)-2',3'-dihydro-indolyl]methyl-2-(3-thienoylamino)]acetyl]amino-5-chlorobenzophenone;

2-N-[2(S)-[2-[3'ß-1'-(Boc-D-leucyl)-2',3'-dihydro-indolyl]methyl-2-(3-thienoylamino)]acetyl]amino-5-chlorobenzophenone;

2-N-[3-(3'-indolyl)propanoyl]amino-5-chloro-benzophenone;

1-($N^{\alpha}$-Boc-L-tryptophanylamino)-2-(N-phenylamino) benzene.

6. A pharmaceutical composition useful for treating gastrointestinal disorders, central nervous system disorders, or regulating appetite in mammals, characterized in that it contains as pharmaceutically effective component at least one compound of formula I according to claim 1 or a pharmaceutically acceptable salt of the compounds of formula I or a quaternary ammonium salt of the compounds of formula I.

7. A pharmaceutical composition according to claim 6 characterized in that it contains as pharmaceutically active ingredient at least one compound of formula I according to claim 2 or a pharmaceutically acceptable salt of said compounds of formula I or an ammonium salt of said compounds of formula I.

8. A pharmaceutical composition according to claim 6 or 7 characterized in that it contains

as active ingredient at least one compound of formula I according to claim 3 or 4, or a pharmaceutically acceptable salt or quaternary ammonium salt of said compound of formula I.

9. Pharmaceutical composition according to one of the patent claims 6 - 8, characterized in that it contains as pharmaceutically active ingredient at least one compound of formula I according to claim 5 or a pharmaceutically acceptable salt or quaternary ammonium salt of said compound of formula I.

10. Pharmaceutical composition according to one of the patent claims 6 - 9, characterized in that it contains as further component a pharmaceutically acceptable carrier.